(19) Europäisches Patentamt
European Patent Office
Office européen des brevets

(11) Veröffentlichungsnummer: **0 064 027**
**B1**

(12) **EUROPÄISCHE PATENTSCHRIFT**

(45) Veröffentlichungstag der Patentschrift: **22.05.85**

(51) Int. Cl.⁴: **C 07 D 317/46,** C 07 D 409/06, A 61 K 31/36

(21) Anmeldenummer: **82810163.4**

(22) Anmeldetag: **19.04.82**

(54) **Neue Benzodioxolderivate, Verfahren zu deren Herstellung und diese enthaltende pharmazeutische Zusammensetzungen.**

(30) Priorität: **24.04.81 CH 2698/81**

(43) Veröffentlichungstag der Anmeldung: **03.11.82 Patentblatt 82/44**

(45) Bekanntmachung des Hinweises auf die Patenterteilung: **22.05.85 Patentblatt 85/21**

(84) Benannte Vertragsstaaten: **AT BE CH DE FR GB IT LI LU NL SE**

(56) Entgegenhaltungen: **DE - A - 2 426 505**

(73) Patentinhaber: **CIBA-GEIGY AG, Postfach, CH-4002 Basel (CH)**

(72) Erfinder: **Habicht, Ernst, Dr., Bienenstrasse 13, CH-4104 Oberwil (CH)**
Erfinder: **Zbinden, Paul, Im Rainacker 6, CH-4108 Witterswil (CH)**

**EP 0 064 027 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Benzodioxolderivate, Verfahren zu deren Herstellung und diese enthaltende pharmazeutische Zusammensetzungen, sowie die Verwendung dieser neuen Stoffe und pharmazeutischen Zusammensetzungen.

In der Deutschen Offenlegungsschrift Nummer 2 426 505 werden 1,3-Benzodioxol-2-carbonsäuren, deren Salze, Ester und Amide, die durch einen gegebenenfalls substituierten Phenylrest am aromatischen Teil des 1,3-Benzodioxolkerns substituiert sind, beschrieben, die sich zur Herabsetzung von Cholesterin und verschiedene Triglyceride enthaltenden Blutlipoproteinen eignen, wogegen die erfindungsgemässen substituierten Arylcarbonyl- und Arylniederalkanoyl-2-benzodioxolcarbonsäuren und deren Derivate diuretische, saliuretische und uricosurische Wirkungen aufweisen.

Die erfindungsgemässen neuen Verbindungen entsprechen der allgemeinen Formel I

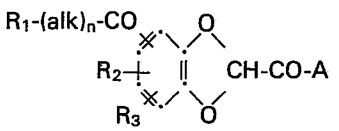

(I)

in welcher $R_1$ einen unsubstituierten oder durch Niederalkyl, Niederalkoxy, Halogen oder Trifluormethyl substituierten aromatischen oder heteroaromatischen Rest, alk einen Alkylen- oder Alkylidenrest mit höchstens 5 Kohlenstoffatomen und n Null oder Eins bedeutet, $R_2$ und $R_3$ unabhängig voneinander Wasserstoff, Niederalkyl, Niederalkoxy oder Halogen bedeuten und A den Rest $-O-R_4$, worin $R_4$ für Wasserstoff oder einen unsubstituierten oder durch Niederalkoxy oder Halogen substituierten aliphatischen oder araliphatischen Kohlenwasserstoffrest steht, oder den Rest

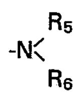

bedeutet, worin $R_5$ und $R_6$ unabhängig voneinander für Wasserstoff oder Niederalkyl, oder unter sich verbunden und zusammen mit dem anliegenden Stickstoffatom für gegebenenfalls niederalkylsubstituiertes Tetra-bis Hexamethyenimino oder 4-Morpholinyl stehen. Sie können als Racemate oder optische Antipoden, oder bei entsprechender Bedeutung der Variablen auch als Racematgemische vorliegen. Ebenfalls Gegenstand der Erfindung sind Salze von Verbindungen der allgemeinen Formel I, in denen A $OR_4$ und darin $R_4$ Wasserstoff bedeutet, mit Basen sowie Säureadditionssalze von Verbindungen der allgemeinen Formel I, deren Rest $R_1$ basischen Charakter aufweist, sowie die Herstellung solcher Salze. Soweit nicht anderes bemerkt, werden vor- und nachstehend unter niederen Resten oder Verbindungen solche mit höchstens 7, vorzugsweise höchstens 4 Kohlenstoffatomen verstanden.

Ein aromatischer Rest $R_1$ ist insbesondere ein 1- oder 2-Naphthylrest und vor allem ein Phenylrest. Ein heteroaromatischer Rest ist vorzugsweise ein bicyclischer und insbesondere ein monocyclischer Rest. Als entsprechender monocyclischer Rest enthält $R_1$ insbesondere zwei Stickstoffatome oder vorzugsweise ein Stickstoffatom und/oder ein Sauerstoff- oder Schwefelatom, und ist beispielsweise ein mono- oder diazacyclischer, oxa- oder thiacyclischer oder oxaza- oder thiazacyclischer Rest mit 5 Ringgliedern, z.B. 1H-Pyrrolyl, wie 1H-Pyrrol-2-yl oder -3-yl, 1H-Pyrazolyl, wie 1H-Pyrazol-3-yl. -4-yl- oder -5-yl, 1H-Imidazolyl, wie 1H-Imidazol-2-yl, -4-yl oder -5-yl, Furyl, wie 2- oder 3-Furyl, Thienyl, wie 2- oder 3-Thienyl, Oxazolyl, wie 2-Oxazolyl, Isoxazolyl, wie 3- oder 5-Isoxazolyl, Thiazolyl, wie 2- oder 4-Thiazolyl, oder ein mono- oder diazacyclischer Rest mit 6 Ringgliedern, z.B. Pyridyl, wie 2-, 3- oder 4-Pyridyl, Pyridazinyl, wie 3-Pyridazinyl, Pyrimidinyl, wie 2-, 4- oder 5-Pyrimidinyl, oder 2-Pyrazinyl. Entsprechende bicyclische Reste $R_1$ bestehen beispielsweise aus einem 5gliedrigen Heteroring aromatischen Charakters mit zwei Stickstoffatom oder mit einem Stickstoffatom und/oder einem Sauerstoff- oder Schwefelatom als Ringgliedern und einem ankondensierten Benzolring, oder aus einem 6gliedrigen Heteroring aromatischen Charakters mit zwei oder insbesondere einem Stickstoffatom als Ringgliedern und einem ankondensierten Benzolring. Dementsprechend ist bicyclisches Heteroaryl z.B. 1H-Indolyl, wie 1H-Indol-2-yl, -3-yl, -4-yl, -5-yl oder -6-yl, 1H-Indazolyl, wie 1H-Indazol-3-y, 1H-Benzimidazolyl, wie 1H-Benzimidazol-2-yl, -4-yl, -5-yl oder -6-yl, Benzofuranyl, wie 2-, 3-, 5- oder 6-Benzofuranyl, Benzo[b]thienyl, wie Benzo[b]thien-2-yl, -3-yl, -5-yl oder -6-yl, Benzoxazolyl, wie 2-, 4-, 5- oder 6-Benzoxazolyl, Benzothiazolyl, wie 2-, 4-, 5- oder 6-Benzothiazolyl, bzw. z.B. Chinolinyl, wie 2-, 4-, 5- oder 6-Chinolinyl, Isochinolinyl, wie 1-, 3- oder 4-Isochinolinyl, Chinazolinyl, wie 2-, 4- oder 6-Chinazolinyl, Chinoxalinyl, wie 2- oder 6-Chinoxalinyl, oder Phthalazinyl, wie 1- oder 6-Phthalazinyl. Als substituierter aromatischer oder heteroaromatischer Rest ist $R_1$ z.B. durch Halogen, wie Fluor, Brom, Jod oder insbesondere Chlor, durch Niederalkyl, wie z.B. Äthyl, Propyl, Isopropyl, Butyl, Isobutyl, tert.-Butyl oder insbesondere Methyl, und/oder Niederalkoxy, wie Äthoxy, Propoxy, Isopropoxy, Butoxy, Isobutoxy und insbesondere Methoxy, und/oder Trifluormethyl ein- oder mehrfach, vorzugs-weise höchstens dreifach, substituiert.

Ein Alkylen- oder Alkylidenrest alk kann gerade oder verzweigt sein und ist z.B. 1,1- bzw. 2,2- oder 1,2-Dimethyläthylen, 1-Äthyläthylen, Tetramethylen oder 1-Propyläthylen bzw. 1-Methylpropyliden, 2-Methylpropyliden, Butyliden oder 1-Äthylpropyliden, insbesondere aber ein Rest mit höchstens 3 Kohlenstoffatomen, wie Äthylen, Propylen, Trimethylen bzw. Äthyliden, Propyliden oder 1-Methyläthyliden und vor allem Methylen.

$R_2$ und $R_3$ sind als Niederalkyl z.B. Äthyl, Propyl, Isopropyl, Butyl, Isobutyl oder tert.-Butyl und insbesondere Methyl; als Niederalkoxy z.B. Äthoxy, Propoxy, Isopropoxy, Butoxy, Isobutoxy und insbesondere Methoxy, und als Halogen Brom, Jod, insbesondere Fluor oder vor allem Chlor. Im Rest A ist $R_4$ als unsubstituierter oder durch Niederalkoxy oder Halogen substituierter aliphatischer oder araliphatischer Kohlenwasserstoffrest z.B. Alkyl- mit höchstens 12 Kohlenstoffatomen, insbesondere Niederalkyl, weiter 2- oder 3-Niederalkenyl oder 2-Niederalkinyl, Niederalkoxyniederalkyl, halogeniertes Niederalkyl, wie geminales Polyhalogenniederalkyl, bzw. z.B. Phenylniederalkyl oder Cinnamyl, in welchen der Phenylrest z.B. in gleicher Weise wie ein Phenylrest $R_1$ substituiert sein kann. Alkyl $R_4$ ist z.B. Propyl, Isopropyl, Butyl, Isobutyl, Pentyl, Isopentyl, Neopentyl, Hexyl, Heptyl, Octyl, Nonyl, Decyl, Dodecyl und vor allem Methyl oder Äthyl; Niederalkenyl $R_4$ z.B. Allyl, 1- oder 2-Methylallyl, 2-Butenyl oder 3-Butenyl; Niederalkinyl z.B. 2-Propinyl; Niederalkoxyniederalkyl insbesondere 2- oder 3-Niederalkoxyniederalkyl, wie z.B. 2-Methoxy-, 2-Äthoxy-, 2-Isopropoxy- oder 2-Butoxyäthyl, 2- oder 3-Methoxypropyl, 2- oder 3-Äthoxypropyl, ferner 3- oder 4-Methoxybutyl oder 3- oder 4-Äthoxybutyl, und halogeniertes Niederalkyl insbesondere geminal, d.h. am gleichen Kohlenstoffatom polyhalogeniertes Niederalkyl, wie 2,2,2-Trifluor- oder 2,2,2-Trichloräthyl. Phenylniederalkyl $R_4$ ist z.B. Benzyl, 2-Phenyläthyl, 2- oder 3-Phenylpropyl oder 2-, 3- oder 4-Phenylbutyl.

Salze der neuen Verbindungen sind insbesondere Salze von Verbindungen der allgemeinen Formel I, worin A Hydroxy bzw. $R_4$ Wasserstoff bedeutet, mit Basen, vor allem pharmazeutisch verwendbare Salze solcher Verbindungen mit Basen. Als solche Salze mit Basen kommen beispielsweise Alkalimetall- oder Erdalkalimetallsalze, wie Natrium-, Kalium-, Calcium- oder Magnesiumsalze, ferner Ammoniumsalze mit Ammoniak oder organischen Aminen, wie Mono- oder Diniederalkylaminen, z.B. Methylamin, Äthylamin, Dimethylamin oder Diäthylamin, oder Mono-, Di- oder Tri(hydroxyalkyl)-aminen, z.B. 2-Aminoäthanol, 2,2'-Iminodiäthanol oder 2,2',2''-Nitrilotriäthanol, in Betracht.

Als Säureadditionssalze, insbesondere pharmazeutisch annehmbare Säureadditionssalze von Verbindungen der allgemeinen Formel I, in denen $R_1$ basischen Charakter aufweist, kommen beispielsweise solche mit geeigneten anorganischen Säuren, wie Halogenwasserstoffsäuren, z.B. Chlorwasserstoffsäure oder Bromwasserstoffsäure, ferner Salpetersäure, Schwefelsäure oder Phosphorsäure, oder mit geeigneten organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organischen Sulfonsäuren, wie gegebenenfalls Hydroxy enthaltenden Niederalkansulfonsäuren, z.B. Methansulfonsäure, Äthansulfonsäure, 2-Hydroxyäthansulfonsäure oder Äthan-1,2-disulfonsäure, oder Arylsulfonsäuren, z.B. Benzolsulfonsäure, 4-Methyl-benzolsulfonsäure oder Naphthalin-2-sulfonsäure, oder weiteren sauren Substanzen, wie Ascorbinsäure, in Frage.

Die neuen Verbindungen der allgemeinen Formel I und ihre Salze besitzen wertvolle pharmakologische Eigenschaften. Sie wirken insbesondere diuretisch und natriuretisch an der Ratte im Dosisbereich von 10 bis 1000 mg/kg per os und am Hund in Dosen ab 20 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei wird die Kalium-Ausscheidung weniger stark gesteigert als die Natrium Ausscheidung; hervorzuheben ist auch die gute Verträglichkeit.

Beispielsweise wird durch die Verabreichung von 10 mg/kg per os 5-[(4-Fluorphenyl)-acetyl]-6-methyl-2-benzodioxolcarbonsäure oder von 10 mg/kg per os 5-Methyl-6-(2-thienylcarbonyl)-2-benzodioxolcarbonsäure an Ratten (3 Tiere pro Dosis) im Vergleich zu unbehandelten Kontrolltieren die Ausscheidung von Natriumionen auf das 3,6fache bzw. 2,6fache, von Kaliumionen auf das 2,2fache bzw. 2,4fache und von Chlorionen auf das 2,6fache bzw. 2,3fache gesteigert. Am Hund wird z.B. durch die Verabreichung von 20 mg/kg per os 5-Benzoyl-6-methyl-2-benzodioxolcarbonsäure (3 Tiere pro Dosis) die durchschnittliche Ausscheidung pro Minute während der ersten 3 oder der ersten 5 Stunden nach Verabreichung im Vergleich zur durchschnittlichen Ausscheidung pro Minute während der letzten Stunde vor Verabreichung bezüglich der Natriumionen auf das 8fache bzw. 5,5-fache, bezüglich der Kaliumionen auf bloss das 1,2fache bzw. gar nicht, bezüglich der Chlorionen auf das 9fache bzw. 6fache und bezüglich des Harnvolumens auf das 2,8fache bzw. 2,2fache gesteigert. Die Kaliumausscheidung wird somit beim Hund durch diese Carbonsäure kaum gesteigert.

Weiter besitzen die Verbindungen der allgemeinen Formel I uricosurische Wirksamkeit, wie sich z.B. anhand von Versuchen am Cebus-Affen (Cebus apella) zeigen lässt. Bei diesen Versuchen wird den Versuchstieren in Pentobarbital-Narkose Polyfructosan in Ringer-Lösung intravenös infundiert und die Prüfsubstanz als wässrige Lösung in ansteigenden Dosen intravenös injiziert. Vor der ersten Prüfsubstanz-Verabreichung und nach jeder Prüfsubstanzdosis wird während je drei 10minütigen Perioden der Harn gesammelt und vor der ersten und nach der letzten Sammelperiode jeweils arteriell Blut entnommen. Die Harnsäure- und Polyfructosan-clearance wird aus deren Plasma- und Urin-Konzentration berechnet und schliesslich die fraktionelle Harnsäureausscheidung (Fractional excretion of uric acid, $FE_{UR}$) als Quotient von Harnsäure-clearance und glomerulärer Filtrationsrate bestimmt. Verbindungen der allgemeinen Formel I zeigen in diesem Test Wirkungen im Dosisbereich von 1 bis 10 mg/kg i.v.; z.B. bewirkt die Verabreichung von 10 mg/kg 5-Benzoyl-6-methyl-2-benzodioxolcarbonsäure nahezu eine Verdopplung der fraktionellen Harnsäureausscheidung. Entsprechend können die Verbindungen der allgemeinen Formel I und ihre pharmazeutisch annehmbaren Salze als kaliumschonende Diuretika mit urikosurischer Zusatzwirkung, z.B. zur Behandlung von Oedemen und der Hypertension, verwendet werden.

Die Erfindung betrifft insbesondere Verbindungen der allgemeinen Formel I, in denen $R_1$ durch Niederalkyl, Niederalkoxy oder Halogen substituiertes oder unsubstituiertes Phenyl, Thienyl oder Furyl, alk Alkylen oder Alkyliden mit höchstens 3 Kohlenstoff-

atomen, $R_2$ Niederalkyl oder Halogen, $R_3$ Wasserstoff, Niederalkyl oder Halogen bedeutet und n und A die unter der Formel I angegebene Bedeutung haben, A jedoch insbesondere $OR_4$ und darin $R_4$ Wasserstoff oder Niederalkyl bedeutet, sowie die Salze von solchen Verbindungen, in denen $R_4$ Wasserstoff ist, mit Basen.

Die Erfindung betrifft ganz besonders Verbindungen der allgemeinen Formel I, in denen $R_1$ durch Niederalkyl, Niederalkoxy oder Halogen substituiertes oder unsubstituiertes Phenyl oder Thienyl, alk Methylen, n Null oder Eins, $R_2$ Niederalkyl, insbesondere Methyl, oder Halogen, insbesondere Fluor oder Chlor, $R_3$ Wasserstoff oder Niederalkyl, insbesondere Methyl, und A $OR_4$ und darin $R_4$ Wasserstoff oder Niederalkyl, bedeutet, sowie die pharmazeutisch annehmbaren Salze von solchen Verbindungen, in denen $R_4$ Wasserstoff ist, mit Basen.

Die Erfindung betrifft vor allem Verbindungen der allgemeinen Formel I, in denen $R_1$ durch Niederalkyl, Niederalkoxy oder Halogen substituiertes oder unsubstituiertes Phenyl, alk Methylen, n Null oder Eins, $R_2$ Niederalkyl, insbesondere Methyl, oder Halogen, insbesondere Fluor oder Chlor, $R_3$ Wasserstoff oder Niederalkyl, insbesondere Methyl, und A $OR_4$ und darin $R_4$ Wasserstoff oder Niederalkyl bedeutet, und der Acylrest vorzugsweise in 5- oder 6-Stellung gebunden ist, sowie die pharmazeutisch annehmbaren Salze von solchen Verbindungen, in denen $R_4$ Wasserstoff ist, mit Basen.

Die Erfindung betrifft in erster Linie Verbindungen der allgemeinen Formel I, in denen $R_1$ durch Niederalkyl, insbesondere Methyl, Niederalkoxy, insbesondere Methoxy, oder Halogen, insbesondere Fluor substituiertes oder vor allem unsubstituiertes Phenyl, alk Methylen, n Eins oder vor allem Null, $R_2$ Niederalkyl, insbesondere Methyl, oder Halogen, insbesondere Fluor oder Chlor, $R_3$ Wasserstoff und A $OR_4$ und darin $R_4$ Wasserstoff oder Niederalkyl bedeuten, und der Acylrest in 5- oder 6-Stellung gebunden ist, sowie die pharmazeutisch annehmbaren Salze von solchen Verbindungen, in denen $R_4$ Wasserstoff ist, mit Basen, wie die drei weiter oben spezifisch genannten Carbonsäuren und ihre pharmazeutisch annehmbaren Salze mit Basen.

Die neuen Verbindungen der allgemeinen Formel I und Salze solcher Verbindungen, in denen A $OR_4$ und darin $R_4$ Wasserstoff ist, oder welche basischen Charakter besitzen, werden in an sich bekannter Weise hergestellt, indem man

a) eine Verbindung der allgemeinen Formel II

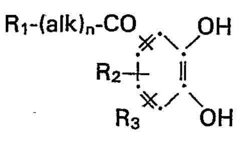

(II)

in welcher $R_1$, alk, n, $R_2$ und $R_3$ die unter der Formel I angegebene Bedeutung haben, oder ein Salz davon mit einer Verbindung der allgemeinen Formel III

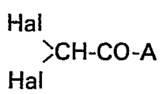

(III)

in welcher Hal Halogen bedeutet und A die unter der Formel I angegebene Bedeutung hat, oder einem Salz einer solchen Verbindung, in der A $OR_4$ und darin $R_4$ Wasserstoff bedeutet, umsetzt, oder

b) in einer Verbindung der allgemeinen Formel IV

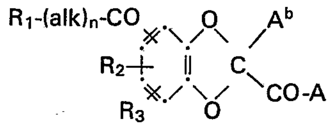

(IV)

in welcher $A^b$ Carboxy, Niederalkoxycarbonyl oder Acetyl bedeutet, und $R_1$, alk, n, $R_2$, $R_3$ und A die unter der Formel I angegebene Bedeutung haben, den Rest $A^b$ durch Wasserstoff ersetzt, oder

c) zur Herstellung einer Verbindung der allgemeinen Formel I, in welcher A die unter der Formel I angegebene Bedeutung hat mit Ausnahme eines Restes $OR_4$, in welchem $R_4$ Wasserstoff bedeutet, und $R_1$, alk, n, $R_2$ und $R_3$ die unter der Formel I angegebene Bedeutung haben, eine Verbindung der allgemeinen Formel V

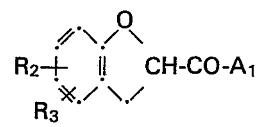

(V)

mit einem Anhydrid einer Verbindung der allgemeinen Formel VI,

$$R_1\text{-}(alk)_n\text{-}CO\text{-}OH \qquad (VI)$$

worin $A_1$ die unter der Formel I für A angegebene Bedeutung hat mit Ausnahme eines Restes $OR_4$, in welchem $R_4$ Wasserstoff bedeutet und $R_2$ und $R_3$ bzw. $R_1$, alk und n die unter der Formel I angegebene Bedeutung haben, umsetzt, oder

d) zur Herstellung einer Verbindung der allgemeinen Formel I, in welcher A $OR_4$ und darin $R_4$ Wasserstoff bedeutet und $R_1$, alk, n, $R_2$ und $R_3$ die unter der Formel I angegebene Bedeutung haben, oder eines Salzes dieser Verbindung, in einer Verbindung der allgemeinen Formel VII

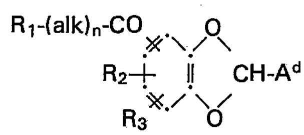

(VII)

in welcher $A^d$ eine in die Carboxygruppe überführbare Gruppe bedeutet und $R_1$, alk, n, $R_2$ und $R_3$ die unter der Formel I angegebene Bedeutung haben, die Gruppe $A^d$ in die Carboxygruppe in freier oder Salzform überführt, oder

e) zur Herstellung einer Verbindung der allgemeinen Formel I, in welcher A die unter der Formel I angegebene Bedeutung hat mit Ausnahme eines Restes $OR_4$, in welchem $R_4$ Wasserstoff bedeutet, und $R_1$, alk, n, $R_2$ und $R_3$ die unter der Formel I angegebene Bedeutung haben, in einer Verbindung der allgemeinen Formel VIII

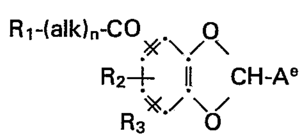

(VIII)

in welcher $A^e$ einen in einen Rest CO-$A_1$, in welchem $A_1$ die unter Formel I für A angegebene Bedeutung hat mit Ausnahme eines Restes $OR_4$, in welchem $R_4$ Wasserstoff bedeutet, überführbaren Rest bedeutet, und $R_1$, alk, n, $R_2$ und $R_3$ die unter der Formel I angegebene Bedeutung haben, den Rest $A^e$ in den Rest -CO-$A_1$ überführt, und gewünschtenfalls eine erhaltene Verbindung der allgemeinen Formel I in an sich bekannter Weise in eine andere Verbindung der allgemeinen Formel I, überführt, und/oder eine als Racemat erhaltene Verbindung der allgemeinen Formel I in die optischen Antipoden zerlegt, und/oder eine erhaltene Verbindung der allgemeinen Formel I, in welcher A $OR_4$ und darin $R_4$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

In den Ausgangsstoffen der allgemeinen Formel III ist Hal vorzugsweise Chlor oder Brom, kann aber auch Fluor oder Jod sein, wobei auch zwei unter sich verschiedene Halogenatome vorliegen können. Die Umsetzungen gemäss Verfahren a) werden vorzugsweise in unter den Reaktionsbedingungen inerten organischen Lösungsmitteln, beispielsweise in ätherartigen, wie z.B. Dibutyläther, 1,2-Dimethoxyäthan, Diäthylenglykoldimethyläther, Tetrahydrofuran oder Dioxan; alkoholartigen, wie z.B. Methanol, Äthanol, Isopropanol, Butanol, 2-Methoxyäthanol oder 2-Äthoxyäthanol; oder amidartigen, wie z.B. Dimethylformamid oder N,N,N',N',N'',N''-Hexamethylphosphorsäuretriamid; oder in Kohlenwasserstoffen, wie z.B. Petroläther, Cyclohexan, Benzol oder Toluol durchgeführt. Umsetzungen mit freien Verbindungen der allgemeinen Formel II wie auch mit freien Halogenessigsäuren der allgemeinen Formel III werden vorzugsweise in Gegenwart von basischen Stoffen durchgeführt. Als solche können beispielsweise organische oder anorganische Derivate von Alkali- oder Erdalkalimetallen, als organische Derivate z.B. Alkalimetall- oder Erdalkalimetallalkoxide, wie Natrium- oder Lithiummethoxid, -äthoxid, -n-butoxid oder -tert.-butoxid, bzw. Barium-methoxid, oder als anorganische Derivate z.B. entsprechende Hydroxide, wie Natrium-, Kalium- oder Calciumhydroxid, oder Carbonate, wie z.B. Natrium- oder Kaliumcarbonat, verwendet werden. Insbesondere Carbonate können in grösserem, z.B. bis fünffachem Überschuss eingesetzt werden. Bei Verwendung von Carbonaten können auch noch weitere organische Lösungsmittel, wie Niederalkanone, z.B. Aceton oder 2-Butanon, als genügend inert in Betracht kommen.

Als Salze von Verbindungen der allgemeinen Formel II und von gegebenenfalls verwendeten, unter die allgemeine Formel III fallenden Dihalogenessigsäuren eignen sich beispielsweise entsprechende Alkalimetallsalze oder Erdalkalimetallsalze. Die Reaktionstemperaturen liegen beispielsweise zwischen Raumtemperatur und etwa 150°C und vorzugsweise zwischen etwa 70 und 120°C.

Eine Anzahl Ausgangsstoffe der allgemeinen Formeln II und III sind bekannt und weitere analog zu den bekannten Verbindungen herstellbar. So kann man beispielsweise Ausgangsstoffe der allgemeinen Formel I herstellen, indem man zunächst Veratrol, das entsprechend der Definition für $R_2$ und $R_3$ substituiert sein kann, mit einem von einer Carbonsäure der Formel $R_1$-COOH oder $R_1$-alk-COOH abgeleiteten Acylhalogenid nach Friedel-Crafts, z.B. mittels Aluminiumchlorid in 1,2-Dichloräthan bei Raumtemperatur, zum entsprechenden Keton kondensiert und in diesem die beiden Methoxygruppen in an sich bekannter Weise, z.B. durch Erhitzen mit Pyridinhydrochlorid oder mit 48%iger Bromwasserstoffsäure in Essigsäure, spaltet. Falls man Ausgangsstoffe der Formel II benötigt, in welchen alk ein Niederalkylidenrest, insbesondere aber ein 1-Niederalkyl-alkyliden wie der 1-Methyläthylidenrest ist, kann man nach der Friedel-Crafts-Kondensation und vor der Spaltung der Methoxygruppen in eine Ketoverbindung, in der alk Methylen oder Niederalkyliden ist, ein oder vorzugsweise zwei Niederalkyl bzw. ein Niederalkyl, insbesondere Methyl, durch Umsetzung mit einem Niederalkylhalogenid, wie Methyljodid, z.B. in einem zweiphasischen System aus einer konzentrierten wässrigen Lösung von Tetrabutylammoniumhydroxid oder -bromid und einem inerten organischen Lösungsmittel, z.B. Methylenchlorid, einführen.

Zur Durchführung des Verfahrens b) erhitzt man beispielsweise einen Ausgangsstoff der allgemeinen Formel IV, in welchem $A^b$ Carboxy bedeutet und A wie auch $R_1$, $R_2$ und $R_3$ die unter der Formel I angegebene Bedeutung haben, in An- oder Abwesenheit eines Katalysators, z.B. Kupferpulver, und/oder eines Lösungs- oder Verdünnungsmittels, wie z.B. o-Dichlorbenzol oder 1,2,3,4-Tetrahydronaphthalin, bis die mindestens annähernd äquimolare Menge Kohlendioxid freigesetzt ist. Ausgangsstoffe der allgemeinen Formel IV, in denen $A^b$ Carboxy und A $OR_4$ und darin $R_4$ Wasserstoff bedeutet, werden beispielsweise durch Hydrolyse von entsprechenden Verbindungen, in denen A $OR_4$ und darin $R_4$ Niederalkyl ist und als bzw. anstelle von $A^b$ Niederalkoxycarbonyl oder Cyano steht, in saurem oder alkalischem Medium, beispielsweise durch Erwärmen mit einer starken Mineralsäure in wässrigem oder wässrig-organischem, z.B. wässrig-niederalkanolischem Medium, bzw. mit der mindestens doppeltmolaren Menge eines Alkalimetallhydroxids, insbesondere Natrium- oder Kaliumhydroxid, z.B. in einem Niederalkanol, wie Methanol, Äthanol, Isopropanol oder n-Butanol, oder in einem Niederalkandiol oder Monoalkyläther desselben, z.B. Äthylenglykol, 2-Methoxyäthanol oder 2-Äthoxyäthanol, hergestellt, wobei den genannten Lösungsmitteln gegebenenfalls Wasser im Volumenverhältnis von Lösungsmittel zu Wasser von ca. 10 : 1 bis 1 : 2 zugefügt wird. Ferner kann als Reaktionsmedium auch Wasser oder z.B. ein Gemisch von Wasser mit wasserlöslichen, ätherartigen Lösungsmitteln, wie Dioxan oder Tetrahydrofuran, verwendet werden.

Erfolgt die Hydrolyse in einer wasserhaltigen Mineralsäure, so kann die verfahrensgemässe Decarboxylierung daran schliessend, d.h. im gleichen Medium und Arbeitsgang durchgeführt werden.

Ausgangsstoffe der allgemeinen Formel IV, in denen $A^b$ Carboxy und A ein Rest entsprechend der

Definition unter der Formel I ist mit Ausnahme eines Restes $OR_4$, in welchem $R_4$ Wasserstoff bedeutet, kann man z.B. durch analoge Hydrolyse in alkalischem Medium von entsprechenden Verbindungen mit Niederalkoxycarbonyl als Rest $A^b$ unter Verwendung der atwa äquimolaren Menge eines Alkalimetallhydroxids anstelle der mindestens doppeltmolaren Menge herstellen. Eine andere Möglichkeit zur Herstellung solcher Ausgangsstoffe der allgemeinen Formel IV besteht in der Hydrogenolyse von entsprechenden Verbindungen, in denen sich anstelle von $A^b$ Benzyloxycarbonyl befindet.

Die Desalkoxycarbonylierung oder Desacetylierung von entsprechenden Ausgangsstoffen der allgemeinen Formel IV, d.h. solchen, in denen $A^b$ Niederalkoxycarbonyl oder Acetyl und A einen definitionsgemässen Rest mit Ausnahme eines Restes $OR_4$, in welchem $R_4$ Wasserstoff ist, bedeutet, erfolgt beispielsweise durch Umsetzung mit der etwa äquimolaren Menge eines Alkalimetallniederalkoxids in einem wasserfreien Niederalkanol, wobei, falls A ein Rest $OR_4$ ist, in welchem $R_4$ Niederalkyl bedeutet, vorzugsweise dasselbe niedere Alkanol, z.B, Methanol, Äthanol, n-Butanol, als Komponente des Ausgangsesters und des Niederalkoxids wie auch als Reaktionsmedium gewählt wird. Man kann aber auch durch Verwendung eines relativ höhersiedenden, mit dem als Esterkomponente vorliegenden Niederalkanol nicht identischen Alkanols als Reaktionsmedium und Abdestillieren eines Teils davon gleichzeitig mit der definitionsgemässen Umsetzung eine Umesterung durchführen oder aber eine partielle Umesterung in Kauf nehmen, falls der als Reaktionsprodukt anfallenden Ester der allgemeinen Formel I nicht direkt als Wirkstoff verwendet, sondern zur entsprechenden Säure hydrolysiert werden soll. Ferner kann als Reaktionsmedium anstelle eines niederen Alkanols z.B. auch ein inertes organisches Lösungsmittel, wie z.B. Benzol oder Toluol, verwendet werden. Die definitionsgemässe Umsetzung wird bei Raumtemperatur oder erhöhter Temperatur, z.B. bei Siedetemperatur des verwendeten Reaktionsmediums, durchgeführt. Gegebenenfalls kann der entstandene Ester der allgemeinen Formel I, wie bereits im Zusammenhang mit der Umesterung erwähnt, im gleichen Arbeitsgang zur entsprechenden Säure hydrolysiert werden, wenn man dem Reaktionsmedium Wasser zufügt.

Die Ausgangsstoffe der allgemeinen Formel IV, in denen $A^b$ Niederalkoxycarbonyl oder Acetyl ist, sowie die weiter oben genannten Vorprodukte zu Verbindungen der allgemeinen Formel IV mit Carboxy als Rest $A^b$, die als bzw. anstelle von $A^b$ Niederalkoxycarbonyl bzw. Cyano enthalten, können analog Verfahren a) durch Umsetzung von Verbindungen der allgemeinen Formel II mit geminalen Dihalogenverbindungen, die sich von solchen der allgemeinen Formel III durch das Vorliegen von Niederalkoxycarbonyl, Acetyl oder Cyano anstelle des neben beiden Halogenatomen befindlichen Wasserstoffatoms unterscheiden, in Gegenwart einer Base hergestellt werden.

Für das Verfahren c) verwendet man als Anhydride von Verbindungen der allgemeinen Formel VI beispielsweise deren Halogenide, wie Chloride oder Bromide, ferner z.B. deren symmetrische Anhydride. Geeignete Katalysatoren für die Umsetzung sind z.B. solche üblicher Friedel-Crafts-Kondensationen, wie Aluminiumchlorid oder Zinn(IV)chlorid, ferner z.B. Zinkchlorid, weiter konz. Schwefelsäure, Phosphorsäure, Polyphosphorsäure oder Pyrophosphorsäure. Die vorgenannten Säuren werden bevorzugt verwendet, wenn man als Derivat einer Carbonsäure der allgemeinen Formel VI ein symmetrisches Carbonsäureanhydrid einsetzt. Die Umsetzung wird vorzugsweise in einem Lösungsmittel vorgenommen. Als solche kann man beispielsweise Halogenkohlenwasserstoffe, wie 1,2-Dichloräthan, Tetrachlorkohlenstoff, Methylenchlorid, o-Dichlorbenzol, weiter z.B. aliphatische oder cycloaliphatische Kohlenwasserstoffe, wie Heptan oder Cyclohexan, Nitrokohlenwasserstoffe, wie Nitromethan, Nitrocyclohexan oder Nitrobenzol und ferner unter milden Bedingungen auch Schwefelkohlenstoff verwenden. Die Reaktionstemperatur liegt zwischen etwa —20° und +80°C, vorzugsweise zwischen etwa 0° und Raumtemperatur.

Die Ausgangsstoffe der allgemeinen Formel V können ihrerseits analog Verfahren a) aus gegebenenfalls entsprechend der Definition für $R_2$ und $R_3$ substituiertem Brenzcatechin, wie z.B. Homobrenzcatechin (4-Methylbrenzcatechin) mit Dihalogenessigsäuren oder deren funktionellen Derivaten entsprechend der allgemeinen Formel III hergestellt werden. Von den als zweite Reaktionskomponenten benötigten funktionellen Derivaten von Verbindungen der allgemeinen Formel VI sind manche bekannt und weitere analog zu den bekannten herstellbar.

Bei der Herstellung von Verbindungen der allgemeinen Formel I, in denen A den Rest $OR_4$ und darin $R_4$ Wasserstoff bedeutet, gemäss Verfahren d) kann die Umwandlung einer Gruppe $A^d$ in die Carboxygruppe in an sich bekannter Weise, insbesondere durch Hydrolyse in alkalischem oder saurem Medium erfolgen, wobei man im ersteren Fall auch direkt ein Salz erhalten kann. Ausgangsstoffe für die Hydrolyse sind zunächst solche Verbindungen der allgemeinen Formel I, in denen A kein Rest $OR_4$ ist, in welchem $R_4$ Wasserstoff bedeutet, besonders leicht hydrolysierbare unter ihnen, wie z.B. die Niederalkylester, im weiteren aber auch andere funktionelle Derivate der als Endstoffe gewünschten Carbonsäuren, wie z.B. Nitrile und Imidoester, insbesondere Imidoniederalkylester, von unter die allgemeine Formel I fallenden Carbonsäuren. Die Hydrolyse erfolgt beispielsweise in niederalkanolischen oder wässrigniederalkanolischen Alkalihydroxidlösungen bei Raumtemperatur bis etwa 100°C bzw. Siedetemperatur des Reaktionsmediums. Niederalkylester, wie Methyl- oder Äthylester und andere leicht spaltbare Ester von unter die allgemeine Formel I fallenden Carbonsäuren können unter noch milderen Bedingungen, z.B. in Gegenwart von Kalium- oder Natriumcarbonat bei Raumtemperatur oder nötigenfalls schwach erhöhen Temperaturen von z.B. 40°C, in wässrig-organischem Medium, z.B. in dem beim Versetzen des bei der Umsetzung gemäss a) erhaltenen Reaktionsgemisches in einem mit Wasser mischbaren Lösungsmittel, wie z.B. 1,2-Dimethoxyäthan, mit Wasser hydrolysiert werden. Aus den dabei zunächst erhaltenen Alkalimetallsalzlösungen der unter die allgemeine Formel I fallenden Carbonsäuren kann man entweder durch Einengen und Abfiltrieren bzw. Eindampfen und Umkristallisieren direkt das entsprechende reine Alkalisalz gewinnen oder zunächst die Carbonsäure freisetzen, anschliessend z.B. durch Umkristallisation reinigen und gewünschtenfalls wiederum in ein Salz mit einer geeigneten anorganischen oder organischen Base überführen. Funktionelle Derivate der unter die allgemeine Formel I fallenden Carbonsäuren lassen sich ferner auch in saurem Medium, z.B. durch Erwärmen in einer mit Wasser verdünnten, z.B. 60-70%igen Schwefelsäure oder in niederalkanolisch-wässeriger Salzsäure, in die freie Carbonsäure der allgemeinen Formel I überführen.

Die benötigten funktionellen Derivate von Carbonsäuren, welche unter die allgemeine Formel I fallen, werden z.B. gemäss Verfahren a), b) oder c), und andere funktionelle Derivate, wie z.B. Nitrile, analog zu diesen Verfahren hergestellt.

Ausgangsstoffe der allgemeinen Formel VIII sind entsprechend dem in ihnen enthaltenden Rest $A^e$ beispielsweise Carbonsäuren, Carbonsäurehalogenide oder -anhydride, insbesondere gemischte Anhydride, weiter aktivierte Ester, z.B. Cyanomethylester, sowie auch Niederalkylester, die, gegebenenfalls in Gegenwart von Kondensationsmitteln, mit Hydroxyverbindungen der allgemeinen Formel IX

$$R_4 - OH \qquad (IX)$$

oder Ammoniak bzw. Aminen der allgemeinen Formel X

$$HN\begin{matrix} R_5 \\ \\ R_6 \end{matrix} \qquad (X)$$

in welchen Formeln $R_4$ bzw. $R_5$ und $R_6$ die unter der Formel I angegebene Bedeutung haben, umgesetzt werden können, oder Salze, insbesondere Alkalimetall- oder Erdalkalimetallsalze der freien Carbonsäuren, die mit reaktionsfähigen Estern von Hydroxyverbindungen der allgemeinen Formel IX, wie Halogeniden oder organischen Sulfonsäureestern, z.B. Niederalkansulfonsäure- oder Arensulfonsäureestern, wie Methansulfonsäure- bzw. p-Toluolsulfonsäureestern, oder auch mit von Aminen der allgemeinen Formel X, deren Reste $R_5$ und $R_6$ von Wasserstoff verschieden sind, abgeleiteten Carbaminsäurehalogeniden, insbesondere -chloriden, umgesetzt werden können; weiter z.B. die zu Estern, insbesondere Niederalkylestern bzw. zu unsubstituierten Amiden hydrolysierbaren Imidoester, insbesondere Imidoniederalkylester, bzw. Nitrile. Freie Carbonsäuren können z.B. auch mit Diazoniederalkanen zu Niederalkylestern oder mit Isocyanaten, die von unter die allgemeine Formel X fallenden primären Aminen abgeleitet sind, zu N-monosubstituierten Amiden, umgesetzt werden.

Die Umsetzungen von freien Carbonsäuren mit Hydroxyverbindungen der allgemeinen Formel IX erfolgen vorteilhaft in Gegenwart eines sauren wasserabspaltenden Katalysators, wie einer Protonensäure, z.B. von Chlor- oder Bromwasserstoff-, Schwefel-, Phosphor- oder Borsäure, Benzolsulfon- oder Toluolsulfonsäure, oder einer Lewissäure, z.B. von Bortrifluorid-Ätherat, in einem Überschuss der eingesetzten Hydroxyverbindung und/oder in einem inerten Lösungsmittel, z.B. in einem Kohlenwasserstoff der Benzolreihe, wie Benzol oder Toluol, einem halogenierten Kohlenwasserstoff wie Chloroform, Methylenchlorid oder Chlorbenzol, der in einem ätherartigen Lösungsmittel, wie Tetrahydrofuran, erforderlichenfalls unter destillativer, z.B. azeotroper, Entfernung des bei der Reaktion freigesetzten Wassers. Weiter kann man die Umsetzungen auch in Gegenwart anderer wasserbindender Kondensationsmittel, z.B. von durch Kohlenwasserstoffreste substituierten Carbodiimiden, wie N,N'-Diäthyl-, N,N'-Dicyclohexyl- oder N-Äthyl-N'-(3-dimethylaminopropyl)-carbodiimid, in inerten organischen Lösungsmitteln, z.B. den vorgenannten, durchführen. Halogenide und gemischte Anhydride werden beispielsweise in Gegenwart säurebindender Mittel, z.B. organischer, insbesondere tertiärer Stickstoffbasen, wie z.B. Triäthylamin, Äthyldiisopropylamin oder Pyridin, oder auch anorganischer Basen, z.B. von Alkalimetall- oder Erdalkalimetallhydroxiden oder -carbonaten, wie Natrium-, Kalium- oder Calciumhydroxid bzw. -carbonat, in inerten organischen Lösungsmitteln, z.B. den obengenannten, und nötigenfalls unter Erwärmen umgesetzt. Die Umsetzungen von reaktionsfähigen Estern von Carbonsäuren der allgemeinen Formel I, z.B. den Cyanomethylestern, mit Hydroxyverbindungen der allgemeinen Formel IX werden beispielsweise in einem gegenüber den Reaktionsteilnehmern inerten Lösungsmittel, z.B. in einem Kohlenwasserstoff wie Toluol oder Xylol, einem ätherartigen Lösungsmittel, wie Tetrahydrofuran oder Dioxan, oder bei mässigen Temperaturen auch einem Ester, wie Äthylacetat, im Temperaturbereich von etwa 0°C bis etwa 120°C, vorzugsweise bei Raumtemperatur bis etwa 60°C durchgeführt. Zu Umesterungen von Niederalkylestern von Carbonsäuren der allgemeinen Formel I verwendet man vorzugsweise Hydroxyverbindungen der allgemeinen Formel IX mit deutlich über demjenigen des veresterten Niederalkanols liegendem Siedepunkt und führt die Reaktion z.B. in einem Überschuss der Hydroxyverbindung und/oder einem inerten organischen, vorzugsweise ebenfalls deutlich über dem Niederalkanol siedenden Lösungsmittel, vorzugsweise in Gegenwart eines Katalysators, z.B. eines Alkalimetallniederalkoxids, wie Natrium- oder Kalium-methoxid oder -äthoxid, in der Wärme und vorzugsweise unter Abdestillieren des freigesetzten Niederalkanols durch. Die Hydrolyse von Imidoestern, insbesondere Imidoniederalkylestern, von Carbonsäuren der allgemeinen Formel I erfolgt beispielsweise mittels wasserhaltiger Mineralsäure, wie Salzsäure oder Schwefelsäure, wobei man z.B. die bei der Addition von Chlorwasserstoff an Nitrile und Umsetzung mit wasserfreien Hydroxyverbindungen der allgemeinen Formel IX insbesonere Niederalkanolen, erhaltenen Imidoester-hydrochloride nach Zusatz von Wasser direkt zu den entsprechenden Estern hydrolysieren kann, oder z.B. auch aus einem Gemisch von Nitril, Hydroxyverbindung und Schwefelsäure von geeignetem Wassergehalt ohne Isolierung des in situ

entstandenen Imidoesters den entsprechenden Ester der allgemeinen Formel I erhalten kann.

Die Umsetzung von freien Carbonsäuren der allgemeinen Formel I mit Verbindungen der allgemeinen Formel X erfolgt beispielsweise in Gegenwart der obengenannten wasserbindenden Mittel und in den dort genannten inerten organischen Lösungsmitteln, man kann aber auch aus den freien Carbonsäuren und den Verbindungen der allgemeinen Formel X zunächst gebildeten Ammoniumsalze durch Erhitzen, gegebenenfalls in einem geeigneten organischen Lösungsmittel von mittlerem oder höherem Siedepunkt, wie z.B. Xylol, Chlorbenzol oder 1,2,3,4-Tetrahydronaphthalin, und destillative, gegebenenfalls azeotropische, Entfernung des bei der Reaktion freigesetzten Wassers, in Amide der allgemeinen Formel I überführen.

Als reaktionsfähige funktionelle Derivate von Carbonsäuren der allgemeinen Formel I zur Umsetzung mit Verbindungen der allgemeinen Formel X und als zugehörige Kondensationsmittel und Lösungsmittel kommen im wesentlichen dieselben in Betracht, wie sie oben für Umsetzungen mit Hydroxyverbindungen der allgemeinen Formel IX angegeben wurden, mit dem Unterschied, dass man als säurebindendes Mittel und gegebenenfalls einziges Reaktionsmedium anstelle anderer, d.h. tertiärer organischer Basen auch einen Überschuss an der umzusetzenden Verbindung der allgemeinen Formel X verwenden kann. Die als weitere Möglichkeit zur Bildung N-unsubstituierter Amide erwähnte partielle Hydrolyse der entsprechenden Nitrile kann beispielsweise mittels wasserhaltiger Mineralsäuren, wie Salzsäure oder verdünnter Schwefelsäure bei Raumtemperatur oder mässig erhöhter Temperatur durchgeführt werden.

Die als Ausgangsstoffe für das Verfahren e) benötigten freien Carbonsäuren der allgemeinen Formel I sind gemäss Verfahren a), b), c) und/oder d), und deren reaktionsfähige funktionellen Derivate. z.B. aus den freien Carbonsäuren in an sich bekannter Weise herstellbar.

Erhaltene salzbildende Verbindungen der Formel I können in an sich bekannter Weise in Salze übergeführt werden, z.B. solche mit Hydroxy als Rest A mit entsprechenden Basen, wie z.B. Alkalimetallhydroxiden, in Salze mit Basen, oder solche von basischem Charakter in ihre Säureadditionssalze. Vorzugsweise werden pharmazeutisch annehmbare Salze hergestellt.

Erhaltene Salze können in an sich bekannter Weise in die freien Verbindungen umgewandelt werden, z.B. durch Behandeln mit einem sauren Reagens, wie einer Mineralsäure, bzw. mit einer Base, z.B. einer Alkalimetallhydroxidlösung, wie Natronlauge.

Die Verbindungen einschliesslich ihrer Salze können auch in Form ihrer Hydrate erhalten werden, oder ihre Kristalle können das zur Kristallisation verwendete Lösungsmittel einschliessen.

Infolge der engen Beziehung zwischen den neuen Verbindungen der allgemeinen Formel I, in welchen A Hydroxy bedeutet, in freier Form und in Form ihrer Salze mit Basen, sowie von solchen Verbindungen, deren Rest $R_1$ basischen Charakter aufweist, in freier Form oder in Form von Säureadditionssalzen, sind im vorausgegangenen und nachfolgend unter den freien Verbindungen oder ihren Salzen sinn- und zweckmässig gegebenenfalls auch die entsprechenden Salze bzw. freien Verbindungen zu verstehen.

Die neuen Verbindungen können, je nach der Anzahl der Asymmetriezentren sowie auch nach der Wahl der Ausgangsstoffe und Arbeitsweisen, in Form von Racematen oder Racematgemischen (Diastereomerengemischen) oder gegebenenfalls auch als reine Antipoden erhalten werden.

Erhaltene Racematgemische können aufgrund der physikalisch-chemischen Unterschiede der Bestandteile in bekannter Weise in die reinen Racemate bzw. Diastereomeren aufgetrennt werden, beispielweise durch Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes der allgemeinen Formel I mit einer mit der racemischen Säure Salze bildenden optisch aktiven Base, bzw. Umsetzung eines basischen Endstoffes der allgemeinen Formel I mit einer optisch aktiven Säure, und Trennung der auf diese Weise erhaltenen Salze, z.B. aufgrund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die Erfindung betrifft im weiteren pharmazeutische Präparate, die Verbindungen der allgemeinen Formel I als Wirkstoffe enthalten, sowie Verfahren zu ihrer Herstellung.

Bei den erfindungsgemässen pharmazeutischen Präparaten handelt es sich um solche zur enteralen, wie oralen oder rektalen, sowie zur parenteralen Verabreichung an Warmblüter. Die Dosierung des Wirkstoffs, der alleine oder zusammen mit dem üblichen Träger- und Hilfsmaterial appliziert wird, hängt von der Warmblüterspezies, deren Alter und dem individuellen Zustand sowie der Applikationsweise ab. Die täglichen Dosen bewegen sich zwischen 0,5 und 30 mg/kg für Mammalien und liegen für solche von etwa 70 kg Gewicht je nach individuellem Zustand und Alter vorzugsweise zwischen 25 und 900 mg, insbesondere zwischen 50 und 600 mg. Entsprechende orale Doseneinheitsformen, z.B. Dragées oder Tabletten oder Kapseln, enthalten vorzugsweise 12,5 bis 300 mg, insbesondere 25 bis 200 mg eines erfindungsgemässen Wirkstoffes, d.h. einer Verbindung

der allgemeinen Formel I oder eines pharmazeutisch annehmbaren Salzes einer zur Salzbildung befähigten Verbindung der allgemeinen Formel I, zusammen mit pharmazeutischen Trägerstoffen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister und Verwendung z.B. von Mais-, Weizen-, Reis- oder Kartoffelstärke, Gelatine, Tragant, Methylcelluloe und/oder Polyvinylpyrrolidin, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten. Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Ölen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit eine Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die Erfindung betrifft ebenfalls die Verwendung der neuen Verbindungen der Formel I und der pharmazeutisch annehmbaren Salze von solchen als pharmakologisch aktive Verbindungen, insbesondere als Diuretika mit urikosurischer Zusatzwirkung, vorzugsweise in Form von pharmazeutischen Präparaten in einem Verfahren zur prophylaktischen und/oder therapeutischen Behandlung des tierischen oder menschlichen Körpers, insbesondere zur Behandlung von Oedemen und/oder der Hypertension.

Die nachfolgenden Beispiele illustrieren die oben beschriebene Erfindung; sie sollen jedoch diese in ihrem Umfang in keine Weise einschränken. Temperaturen werden in Celsiusgraden angegeben.

*Beispiel 1*

Zu einer Lösung von 20,8 g (0,1 Mol) 5-Methyl-2-benzodioxolcarbonsäure-äthylester und 16,9 g (0,12 Mol) Benzoylchlorid in 250 ml 1,2-Dichloräthan gibt man unter Rühren bei 5 bis 10° innerhalb 30 Minuten portionenweise 33,3 g (0,25 Mol) Aluminiumchlorid, rührt das Gemisch noch 30 Minuten bei 5 bis 10° und erwärmt es dann auf Raumtemperatur. Hierauf wird es auf ein Gemisch von etwa 500 g Eis und etwa 1000 ml Wasser gegossen. Aus der zunächst entstandenen Emulsion scheidet sich allmählich die organische Phase ab. Diese wird abgetrennt und nacheinander mit zweimal je 100 ml Wasser, einmal 200 ml 2-n.Natriumcarbonatlösung und zweimal 100 ml Wasser gewaschen. Die vereinigten wässerigen Phasen werden ihrerseits zweimal mit je 100 ml Chloroform nachextrahiert und der Rückstand dieser Extrakte mit der organischen Phase vereinigt. Letztere wird mit Natriumsulfat getrocknet, filtriert und eingedampft. Der zurückbleibende rohe 5-Benzoyl-6-methyl-2-benzodioxolcarbonsäure-äthylester wird durch Säulenchromatographie an 1000 g Kieselgel unter Verwendung von Chloroform-Petroläther-Äthylacetat im Verhältnis 10 : 10 : 1 als Lösungs- und Eluiermittel gereinigt. Nach Vorlauffraktionen von etwa 7 bis 10% der Gesamtmenge erhält man den obengenannten Ester als Hauptprodukt von zähflüssiger Konsistenz, das direkt weiterverwendet werden kann.

14,2 g (0,05 Mol) 5-Benzoyl-6-methyl-2-benzodioxolcarbonsäure-äthylester werden in 160 ml Äthanol gelöst, 120 ml 2-n.Natronlauge zugegeben und

das Gemisch 30 Minuten bei Raumtemperatur gerührt. Dann wird das Äthanol bei vermindertem Druck abgedampft, die verbleibende alkalische Lösung mit wenig Bleicherde versetzt und genutscht. Das Filtrat wird mit 150 ml 2-n.Salzsäure versetzt, die ausgeschiedene Säure mit etwa 800 ml Äthylacetat extrahiert und anschliessend noch zweimal mit je 100 ml Äthylacetat extrahiert. Die organischen Phasen werden vereinigt, zweimal mit je 100 ml Wasser gewaschen, mit Natriumsulfat getrocknet, filtriert und eingedampft. Der Rückstand wird in wenig Äther gelöst und die Lösung bis zur Trübung mit Petroläther versetzt. Hiebei scheidet sich eine Verunreinigung ab, die nach Zugabe von etwas Bleicherde abfiltriert wird. Das klare gelbe Filtrat wird eingedampft, in 25 ml Äthylacetat gelöst und erneut filtriert. Das Filtrat wird bis zur Trübung mit Cyclohexan versetzt und mit der gewünschten Säure, falls vorhanden, angeimpft, wobei die 5-Benzoyl-6-methyl-2-benzodioxolcarbonsäure vom Smp. 119-122° erhalten wird. Aus dem Mutterlaugenrückstand kann durch Lösen in Äthylacetat und Versetzen mit Cyclohexan wie oben weiteres Reaktionsprodukt gewonnen werden.

Der als Ausgangsstoff verwendete 4-Methyl-2--benzodioxolcarbonsäure-äthylester wird wie folgt hergestellt:

a) In eine unter trockenem, sauerstofffreiem Stickstoff und Wasserausschluss in üblicher Weise durch Auflösen von 46,0 g (2 Mol) Natrium in 1500 ml abs. Äthanol bereiteten Natriumäthoxidlösung werden bei etwa 15° innerhalb 10 Minuten 124 g (1 Mol) Homobrenzcatechin (4-Methylbrenzcatechin) eingetragen, wobei eine dunkle, an der Oberfläche blaue Lösung entsteht. Hierauf werden bei etwa 10° innerhalb 20 Minuten 157 g (1 Mol) Dichloressigsäure-äthylester zugetropft. Das Reaktionsgemisch wird anschliessend eine Stunde bei Raumtemperatur gerührt und dann 6 Stunden unter Rückfluss gekocht. Zu der entstandenen dunkelbraunen Lösung gibt man unter Rühren 10 g «Kieselgel H nach Stahl» und filtriert durch Diatomeenerde. Das dunkelbraune Filtrat wird im Vakuum eingedampft, der schwarze, dickflüssige Rückstand in genügend Äther und 300 ml 5%iger Natriumbicarbonatlösung gelöst, die Ätherphase zuerst mit Natriumbicarbonatlösung und dann, zur Entfernung von noch vorhandenem Homobrenzcatechin 6mal mit je 50 ml 2-n.Natronlauge extrahiert und schliesslich zweimal mit Wasser gewaschen, über Natriumsulfat getrocknet filtriert und im Rotationsverdampfer eingedampft. Die zurückbleibende rote Flüssigkeit wird im Vakuum destilliert, wobei der gewünschte 5-Methyl-2-benzodioxolcarbonsäure-äthylester unter 26 mbar bei 155-158° übergeht.

Der 5-Methyl-2-benzodioxolcarbonsäure-äthylester kann aus Homobrenzcatechin auch in zwei Stufen wie folgt hergestellt werden:

b) In 1000 ml 1,2-Dimethoxyäthan löst man unter Rühren in Stickstoffatmosphäre nacheinander 186,2 g (1,5 Mol) Homobrenzcatechin und 263,3 g (184,3 ml, 1,5 Mol) Dichloressigsäureäthylester und fügt anschliessend portionenweise 1040,2 g (7,54 Mol) pulverisiertes Kaliumcarbonat zu, wobei die Temperatur auf über 50° steigt und eine dicke Suspension entsteht. Dieses Reaktionsgemisch wird 5 Stunden unter Rühren zum Rückfluss erhitzt (Badtemperatur), dann auf 40° abgekühlt und langsam mit ca. 2000 ml Wasser versetzt. Hierbei entsteht eine braune Emulsion, die auch Festsubstanz enthält. Am Rotationsverdampfer wird das 1,2-Dimethoxyäthan abdestilliert und die zurückbleibende schwarze Lösung mit Bleicherde versetzt und durch Diatomeenerde filtriert. Das dunkelbraune Filtrat wird mit konz. Salzsäure auf den pH-Wert 2 eingestellt, wobei unter starkem Schäumen die rohe Säure als Harz ausfällt. Das Gemisch wird dreimal mit Äthylacetat extrahiert, die vereinigten Äthylacetatlösungen werden zweimal mit Wasser gewaschen, über Natriumsulfat getrocknet und eingedampft, wobei hellbraune, harzbedeckte Kristalle zurückbleiben. Diese werden in etwa 700 ml Äthylacetat gelöst und die schwarze Lösung mit 1400 ml gesättigter Natriumbicarbonatlösung gemischt, wobei unter leichtem Schäumen eine Emulsion entsteht, aus der sich die wässrige Phase wieder absetzt. Sie wird zweimal mit je 300 ml Äthylacetat extrahiert und die Äthylacetatlösungen ihrerseits zweimal mit je 500 ml Wasser extrahiert. Die wässrigen Phasen werden mit der oben erhaltenen vereinigt, mit etwa 130 ml konz. Salzsäure auf einen pH-Wert von 1 bis 2 eingestellt und 3mal mit Äther extrahiert. Die Ätherlösungen werden je zweimal mit Wasser gewaschen vereinigt, mit Natriumsulfat getrocknet, mit Bleicherde versetzt, filtriert und eingedampft. Das erhaltene braune Öl wird zur Entfernung von noch vorhandenem Harz dann mit 200 und noch 6mal mit je 100 ml warmem Cyclohexan extrahiert, wobei die Portionen jeweils abdekantiert werden. Beim Abkühlen der vereinigten Portionen erhält man die 5-Methyl-2-benzodioxolcarbonsäure in Form weisser Kristalle vom Smp. 108-110°.

c) 104,1 g (0,5 Mol) 5-Methyl-2-benzodioxolcarbonsäure werden in 1000 ml abs. Äthanol, das 0,5 ml konz. Salzsäure enthält, 15 Minuten unter Rückfluss gekocht. Dann wird die klare gelbe Lösung eingedampft und der Rückstand in einem Gemisch von Äther und 1-n.Natriumbicarbonatlösung gelöst. Die Ätherphase wird zweimal mit Wasser extrahiert und die vereinigten wässrigen Phasen ihrerseits noch zweimal mit Äther extrahiert. Die vereinigten Ätherphasen werden über Natriumsulfat getrocknet, filtriert und eingedampft, wobei der rohe 5-Methyl-2--benzodioxolcarbonsäure-äthylester als braunes Öl zurückbleibt. Bei der anschliessenden Destillation im Vakuum geht er unter etwa 21 mbar bei 152-154° über. Als Ausgangsstoff können anstelle der weissen Substanz vom Smp. 108-110° auch die unter b) erhaltenen harzbedeckten Kristalle verwendet werden.

*Beispiel 2*

Zu einer Lösung von 31,2 g (0,15 Mol) 5-Methyl--2-benzodioxolcarbonsäure-äthylester und 28,5 g (21,3 ml, 0,18 Mol) 2-Fluorbenzoylchlorid in 350 ml 1,2-Dichloräthan werden bei 5° bis 10° unter Rühren 46,6 g (0,35 Mol) Aluminiumchlorid innerhalb 25 Minuten portionenweise zugegeben. Hierauf wurde das Reaktionsgemisch noch 90 Minuten bei 5-10° und 30 Minuten bei Raumtemperatur gerührt, worauf alles Aluminiumchlorid aufgelöst ist. Nach ca. 24 Stunden stehenlassen bei Raumtemperatur wird das Reaktionsgemisch auf ca. 2 kg Eis-Wasser-Gemisch gegossen. Die organische Phase wird abgetrennt, einmal mit 2-n.Salzsäure, zweimal mit Wasser, einmal mit 1-n.Natriumbicarbonatlösung und noch zweimal mit Wasser gewaschen, dann über Natriumsulfat getrocknet und im Vakuum eingedampft. Der als Rückstand erhaltene rohe 5-(2-Fluorbenzoyl)-6-methyl-2-benzodioxolcarbonsäure-äthylester kann direkt weiterverarbeitet werden.

59 g (0,15 Mol) 5-(2-Fluorbenzoyl)-6-methyl-2--benzodioxolcarbonsäure-äthylester werden in 270 ml Äthanol gelöst und bei Raumtemperatur 130 ml 2-n.Natronlauge zugegeben, wobei Dunkelfärbung und leichte Erwärmung eintritt. Nach 2 Stunden stehenlassen bei Raumtemperatur wird das Äthanol im Vakuum abdestilliert, der Rückstand mit ca. dem halben Volumen Wasser versetzt und zweimal mit Äther extrahiert. Hierauf wird die wässrige Lösung mit ca. 50 ml 6-n.Salzsäure angesäuert und die ausgeschiedene Säure mit Äthylacetat extrahiert. Die organische Phase wird zweimal mit Wasser gewaschen, getrocknet und eingedampft, wobei die rohe 5-(2-Fluorbenzoyl)-6-methyl-2-benzodioxolcarbonsäure als sandfarbenes Pulver zurückbleibt. Zur Reinigung wird sie in wenig Äthylacetat gelöst, das gleiche Volumen Cyclohexan zugegeben, dann die heisse Lösung mit Bleicherde versetzt und durch diatomeenerde filtriert. Das Filtrat wird im Rotationsverdampfer eingeengt, bei Raumtemperatur stehengelassen und schliesslich auf ca. 10° gekühlt. Die Kristalle werden abfiltriert, mit wenig Äthylacetat-Cyclohexan und nochmals aus Äthylacetat-Cyclohexan umkristallisiert. Die so erhaltene Substanz schmilzt bei 152 bis 156°.

*Beispiel 3*

Analog Beispiel 2 erhält man unter Verwendung von 28,5 g (0,18 Mol) 4-Fluorbenzoylchlorid den 5--(4-Fluorbenzoyl)-6-methyl-2-benzodioxolcarbonsäure-äthylester als öliges Rohprodukt, und
aus 16,5 g (0,05 Mol) des vorgenannten Produktes die 5-(4-Fluorbenzoyl)-6-methyl-2-benzodioxolcarbonsäure vom Smp. 93-94° (aus Äthylacetat).

*Beispiel 4*

Analog Beispiel 2 erhält man unter Verwendung von 34,1 g (0,2 Mol) 4-Methoxybenzoylchlorid den 5-(4-Methoxybenzoyl)-6-methyl-2-benzodioxolcarbonsäure-äthylester, und
aus 17,1 g (0,05 Mol) des obigen Produktes die 5-(4--Methoxybenzoyl)-6-methyl-2-benzodioxolcarbonsäure vom Smp. 55-57° (aus Äthylacetat-Petroläther).

*Beispiel 5*

Analog Beispiel 2 erhält man unter Verwendung von 31,2 g (0,18 Mol) (4-Fluorphenyl)-acetylchlorid den 5-[(4-Fluorphenyl)-acetyl]-6-methyl-2-benzodioxolcarbonsäure-äthylester als öliges Rohprodukt, und
aus 17,2 g (0,05 Mol) des obigen Produktes die 5-[(4-Fluorphenyl)-acetyl]-6-methyl-2-benzodioxolcarbonsäure vom Smp. 106-109° (aus Äthylacetat-Cyclohexan).

*Beispiel 6*

Zu einer Lösung von 20,8 g (0,1 Mol) 5-Methyl-2--benzodioxolcarbonsäure-äthylester und 17,7 g (0,12 Mol) 2-Thiophencarbonylchlorid in 250 ml 1,2-Dichloräthan werden unter Rühren bei 5 bis 8° innerhalb von 30 Minuten 33,3 g (0,25 Mol) Aluminiumchlorid portionenweise zugegeben. Nach weiteren 30 Minuten Rühren bei 5° bis 8° wird das Kühlbad entfernt und das Reaktionsgemisch noch 3 Stunden bei Raumtemperatur gerührt. Hierauf werden noch 100 ml 1,2-Dichloräthan zugefügt und das Reaktionsgemisch auf ein Gemisch von 500 g Eis und 1000 ml Wasser gegossen. Die organische Phase wird abgetrennt und nacheinander zweimal mit je 100 ml Wasser, einmal mit 200 ml 2-n.Natriumcarbonatlösung und noch zweimal mit je 100 ml Wasser gewaschen. Andererseits wird die wässrige Phase zweimal mit je 100 ml Chloroform extrahiert und der Rückstand dieser Extrakte mit der organischen Phase vereinigt. Letztere wird mit Natriumsulfat getrocknet, filtriert und im Vakuum eingedampft. Der Rückstand wird an einer Säule von 1000 g Kieselgel unter Verwendung von Chloroform-Petroläther-Äthylacetat im Verhältnis von 10 : 10 : 1 als Lösungs- und Eluiermittel chromatographiert. Nach etwa 10% der Gesamtmenge an Vorlaufprodukten wird der gewünschte 5-Methyl-5-[(2-thienyl)-carbonyl]-2-benzodioxolcarbonsäure-äthylester als Hauptprodukt von etwa 80% eluiert. Die entsprechenden Fraktionen kristallisieren spontan und können direkt für die nächste Stufe verwendet werden. Eine Probe wird mit wenig Petroläther und Äthylacetat versetzt, und die erhaltene Kristallsuspension filtriert. Die so erhaltenen Kristalle des obigen Esters schmelzen bei 69-70°.

15,9 g (0,05 Mol) 5-Methyl-6-[(2-thienyl)-carbonyl]-2-benzodioxolcarbonsäure-äthylester löst man in 160 ml Äthanol unter Erwärmen auf etwa 60° und gibt zu der wieder auf Raumtemperatur gekühlten Lösung unter Rühren 75 ml 2-n.Natronlauge. Nach 15 Minuten Rühren bei etwa 20° wird das Äthanol im Vakuum abgedampft, die zurückbleibende Lösung mit wenig Bleicherde versetzt, filtriert und 90 ml 2-n.Salzsäure versetzt. Die ausgeschiedene Säure wird mit etwa 300 ml Äthylacetat extrahiert, die Äthylacetatlösung zweimal mit je 150 ml Wasser gewaschen und die wässrigen Phasen nochmals mit Äthylacetat extrahiert. Die vereinigten organischen Phasen werden mit Natriumsulfat getrocknet, filtriert und eingeengt, wobei das gewünschte Produkt kristallisiert. Es wird abfiltriert und aus Äthylacetat umkristallisiert. Die so erhaltene 5-Methyl-6-[(2--thienyl)-carbonyl]-2-benzodioxolcarbonsäure schmilzt bei 136-137°.

*Beispiel 7*

Ein Gemisch von 4,05 g (0,015 Mol) 1-(4,5-Dihydroxy-2-methylphenyl)-2-methyl-2-phenyl-1-propanon, 2,83 g (0,018 Mol) Dichloressigsäure-äthylester und 5,6 g (0,04 Mol) wasserfreiem Kaliumcarbonat wird in 15 ml Dimethylformamid zwei Stunden bei 90° unter Stickstoffatmosphäre gerührt. Hierauf wird das Dimethylformamid im Rotationsverdampfer so weit als möglich abgedampft. Der Rückstand wird in 100 ml Wasser gelöst und die Lösung eine Stunde

bei Raumtemperatur stehengelassen. Hierauf wird sie mit verdünnter Salzsäure kongosauer gestellt, die freigesetzte 5-Methyl-6-(2-methyl-2-phenylpropionyl)-2-benzodioxolcarbonsäure mit Äthylacetat extrahiert und die erhaltene Lösung eingedampft. Durch Umkristallisieren des Rückstandes erhält man die reine Carbonsäure als farblose Nädelchen vom Smp. 117-119°.

Das benötigte Dihydroxyketon wird wie folgt hergestellt:

a) In eine Lösung von 84,1 g (0,5 Mol) 1,2-Dimethoxy-4-methylbenzol (Homobrenzcatechindimethyläther bzw. 4-Methylveratrol) in 750 ml 1,2-Dichloräthan werden unter Rühren 80,0 g (0,6 Mol) Aluminiumchlorid portionsweise eingetragen, wobei man durch Kühlen mit einem Eisbad die Temperatur bei höchstens 20° hält. Dann werden bei 15-20° innerhalb einer Stunde 85,0 g (ca. 73 ml, 0,55 Mol) Phenylacetylchlorid zugetropft, wobei mässige Chlorwasserstoffentwicklung eintritt und das Aluminiumchlorid aufgelöst wird.

Hierauf wird das Reaktionsgemisch noch 6 Stunden bei Raumtemperatur gerührt und anschliessend auf 3000 ml Eis-Wasser-Gemisch gegossen. Die entstandenen Schichten werden getrennt und die organische Phase nacheinander mit 2-n.Salzsäure, zweimal mit Wasser, dann mit 1-n.Natriumbicarbonatlösung und noch zweimal mit Wasser gewaschen, getrocknet, filtriert und eingedampft. Der zum Teil kristallisierte Rückstand wird aus Äther-Petroläther (Siedebereich 40-65°) vollständig zur Kristallisation gebracht, wobei das 1-(4,5-Dimethoxy-2-methylphenyl)-2-phenyl-1-äthanon vom Smp. 45-48° erhalten wird.

b) Eine Lösung von 13,5 g (0,05 Mol) 1-(4,5-Dimethoxy-2-methyl-phenyl)-2-phenyl-1-äthanon und 21,3 g (9,35 ml, 0,15 Mol) Methyljodid in 135 ml Methylenchlorid wird innerhalb 45 Minuten unter Rühren bei Raumtemperatur zu 100 ml einer 40%-igen Lösung von Tetrabutylammonium-hydroxid (etwa 0,15 Mol) zugetropft. Eine mässig exotherme Reaktion erwärmt dabei das Reaktionsgemisch auf etwa 35°. Anschliessend wird das Reaktionsgemisch noch 10 Stunden ohne Erwärmen oder Kühlen gerührt. Dann werden die Phasen getrennt, die organische Phase zweimal mit Wasser gewaschen, über Natriumsulfat getrocknet, filtriert und eingedampft. Der Rückstand wird mit 300 ml Äther versetzt, wobei das Tetrabutylammoniumjodid auskristallisiert. Es wird abfiltriert und das Filtergut mit Äther nachgewaschen. Das Filtrat wird eingedampft und der obige Rückstand in einem Gemisch von Petroläther (Siedebereich 40-60°), Chloroform und Äthylacetat 10 : 10 : 1 gelöst und an 450 g Kieselgel adsorbiert. Durch Eluieren mit dem vorgenannten Gemisch und Eindampfen der ersten 4 Liter Eluat erhält man das 1-(4,5-Dimethoxy-2-methylphenyl)-2-methyl-2-phenyl-1-propanon als Öl, das direkt weiterverwendet werden kann.

c) 8,0 g des öligen Produktes von b) (etwa 0,026 Mol) werden mit 50 g Pyridin-hydrochlorid gemischt und das Gemisch 4 Stunden auf 200° (Innentemperatur) erhitzt. Hierauf lässt man das Reaktionsgemisch sich abkühlen, versetzt es mit Eis und 50 ml 2-n.Salzsäure und extrahiert das ausgeschiedene Reaktionsprodukt mit Äthylacetat. Durch Eindampfen der erhaltenen Lösung und Umkristallisieren des teilweise kristallisierten Rückstandes aus Äthylacetat-Cyclohexan gelangt man zum 1-(4,5-Dihydroxy-2-methylphenyl)-2-methyl-2-phenyl-1-propanon vom Smp. 189-191°.

*Beispiel 8*

Eine Lösung von 71,0 g (0,24 Mol) 5-Benzoyl-6-methyl-2-benzodioxolcarbonsäure (Smp. 120 bis 122°, vgl. Beispiel 1) in 200 ml Acetonitril und eine Lösung von 41,2 g (0,25 Mol) D-Ephedrin (Base) in 300 ml Acetonitril von je etwa 30° werden zusammengefügt und im Eisbad abgekühlt und die dabei sich abscheidenden Kristalle werden abfiltriert. Die erhaltene Kristallfraktion wird viermal aus Acetonitril umkristallisiert, wobei man das D-Ephedrinsalz der (+)-5-Benzoyl-6-methyl-2-benzodioxolcarbonsäure als farblose, feine Nadeln vom Smp. 149-150° (Sintern ab 145°) erhält.

Durch Lösen in Wasser, Ansäuern mit 2-n.Salzsäure und Abfiltrieren erhält man die entsprechende freie Säure, welche nach Kristallisation aus 50 ml Äthylacetat unter Zusatz von 20 ml Petroläther (Siedebereich 40-60°), bei 145-147° schmilzt; $[\alpha]_D^{20}$ + 67° (c = 1% in Aceton).

Aus den Mutterlaugen-Rückständen der obigen Kristallisationen wird die Carbonsäure freigesetzt und abgetrennt und in analoger Weise als Lösung in Acetonitril mit der entsprechenden Lösung von L-Ephedrin in Acetonitril umgesetzt. Das dabei als Rohrprodukt erhaltene Salz des L-Ephedrins mit der (—)-5-Benzoyl-6-methyl-2-benzodioxolcarbonsäure wird ebenfalls analog zunächst viermal aus Acetonitril umkristallisiert, und dann aus dem erhaltenen reinen Salz vom Smp. 149-150° (Sintern ab 145°) die Carbonsäure freigesetzt. Smp. der letzteren nach Umkristallisation aus Äthylacetat/Phenoläther (s. oben) 145-147°C, $[\alpha]_D^{20}$ — 67° ± 1° (c = 1% in Aceton).

*Beispiel 9*

Eine Suspension von 46,7 g (0,338 Mol) frisch ausgeglühtem Kaliumcarbonat in 180 ml 1,2-Dimethoxyäthan wird unter Stickstoff mit einem hochtourigen Rührwerk 10 Minuten gerührt. Dann fügt man unter normalem Rühren 15,7 g (67,6 mMol) (4,5-Dihydroxy-2-fluorphenyl)-phenylmethanon und 10,6 g (67,6 mMol) Dichloressigsäure-äthylester hinzu und heizt das Gemisch auf 70°. Nach 6 Stunden gibt man noch 3,9 g (20 mMol) Dichloressigsäure-äthylester zu und kocht das Gemisch weitere 15 Stunden. Dann wird es mit Wasser versetzt, mittels Salzsäure auf pH 1-2 eingestellt, unter Vakuum teilweise eingedampft und mit Äthylacetat extrahiert. Die organischen Phasen werden mit Wasser und gesättigter Natriumchloridlösung gewaschen, über Natriumsulfat getrocknet und vollständig eingedampft. Der Rückstand wird mit Äthanol/p-Toluolsulfonsäure verestert. Der Ester wird durch Chromatographie an Kieselgel unter Elution mit Äthylacetat/n-Hexan-Gemisch (Verhältnis 1 : 1) gereinigt. Nach Eindampfen der entsprechenden Fraktionen wird der Ester in Methanol gelöst und mit 1-n.Natronlauge bei Raumtemperatur verseift. Das Reaktionsgemisch wird mit Eis/Wasser versetzt, angesäuert

und mit Äthylacetat extrahiert. Die vereinigten Äthylacetatextrakte werden gewaschen, getrocknet und eingedampft. Der gelbe Rückstand wird mit n-Hexan aufgekocht und abgenutscht. Nach zwei Umkristallisationen des Filtergutes aus 1,2-Dichloräthan erhält man die 5-Benzoyl-6-fluor-2-benzdioxolcarbonsäure als weisse Kristalle vom Smp. 152 bis 153,5°.

Das benötigte (4,5-Dihydroxy-2-fluorphenyl)-phenylmethanon kann wie folgt hergestellt werden:

a) Sechs Bombenrohre, jedes 4,8 g (30 mMol) 4-Fluor-1,2-dimethoxybenzol (aus 3,4-Dimethoxyanilin durch eine Balz-Schiemann-Reaktion erhalten), 7,5 g (37,5 mMol) (2-Pyridinyl)-benzoat und 300 ml Trifluoressigsäure enthaltend, werden unter magnetischem Rühren 4 1/2 Stunden auf 100° erhitzt. Der Inhalt aller sechs Bombenrohre wird unter starkem Rühren in 600 ml Wasser gegossen, und die ausgeschiedene feste Substanz wird abgenutscht. Das Filtrat wird mehrmals mit Toluol extrahiert. Die feste Substanz wird in den vereinigten Toluolextrakten gelöst. Die Toluollösung wird mit Wasser und gesättigter Natriumchloridlösung gewaschen, über Magnesiumsulfat getrocknet und zur Trockne eingedampft. Das erhaltene halbfeste Öl wird in 500 ml warmem Isopropanol gelöst und mit Kohle behandelt. Die filtrierte Lösung wird unter Vakum bis zum Kristallisationsbeginn eingeengt. Nach völliger Kristallisation wird das (4,5-Dimethoxy-2-fluorphenyl)-phenylmethanon abgenutscht und mit kaltem Isopropanol gewaschen, wobei man weisse Kristalle vom Smp. 102-104° erhält.

Man kann dieselbe Substanz auch wie folgt herstellen:

b) Unter Stickstoff rührt man 12,7 g (95 mMol) Aluminiumtrichlorid in 80 ml 1,2-Dichloräthan und kühlt bis 3°. Man fügt 13,35 g (95 mMol) Benzoylchlorid hinzu. Diese rote Lösung wird innerhalb 25 Minuten bei 3° in eine Lösung von 12,55 g (80,3 mMol) 1,2-Dimethoxy-4-fluorbenzol (4-Fluorveratrol) in 80 ml 1,2-Dichloräthan eingetropft. Das Reaktionsgemisch wird 3 Stunden bei 3°, dann 3 1/2 Stunden bei Raumtemperatur weitergerührt und dann auf das Gemisch von 250 ml Eis und 20 ml konz. Salzsäure gegossen. Die organische Phase wird abgetrennt und die wässrige Phase mehrmals mit Äther extrahiert. Organische Phase und Ätherlösungen werden vereinigt, mit 1-n.Natronlauge, Wasser und mit gesättigter Natriumchloridlösung gewaschen, über Magnesiumsulfat getrocknet und eingedampft. Das erhaltene gelbe Öl wird mit 50 ml Äther im Eisbad bis zur Kristallisation verrührt, wobei man das gewünschte (4,5-Dimethoxy-2-fluorphenyl)-phenylmethanon als weisse Kristalle vom Smp. 102 bis 103° erhält.

Zur Demethylierung kocht man ein Gemisch von 21,0 g (80,7 mMol) (4,5-Dimethoxy-2-fluorphenyl)-phenylmethanon, 80 ml Eisessig und 80 ml Bromwasserstoffsäure (48%) 17 Stunden unter Rückfluss. Dann wird das Reaktionsgemisch auf Eis/Wasser gegossen und mehrmals mit Äthylacetat extrahiert. Die vereinigten Extrakte werden mit Wasser und gesättigter Natriumchloridlösung gewaschen, mit Aktivkohle behandelt, über Natriumsulfat getrocknet und zur Trockene eingedampft. Der Rückstand wird zweimal aus 1,2-Dichloräthan umkristallisiert, wobei das (4,5-Dihydroxy-2-fluorphenyl)-phenylmethanon vom Smp. 169,5-171° erhalten wird.

*Beispiel 10*

Eine Suspension von 83 g (0,60 Mol) frisch ausgeglühtem Kaliumcarbonat in 250 ml 1,2-Dimethoxyäthan wird unter Stickstoff 10 Minuten mit einem hochtourigen Rührwerk gerührt. Dann gibt man unter normalem Rühren 28,10 g (0,113 Mol) (4,5-Dihydroxy-2-chlorphenyl)-phenylmethanon und 19,5 g (0,124 Mol) Dichloressigsäure-äthylester zu. Das Reaktionsgemisch wird 20 Stunden unter Rückfluss gekocht, dann mit 400 ml Wasser versetzt und eine weitere Stunde gerührt. Ein Teil des Lösungsmittels wird unter Vakuum abgedampft und die Wasserphase mehrmals mit Äthylacetat extrahiert. Die organischen Extrakte werden vereinigt, mit Wasser und gesättigter Natriumchloridlösung gewaschen, mit Aktivkohle behandelt, über Magnesiumsulfat getrocknet und eingedampft. Das erhaltene braune Öl wird an Kieselgel mit Hilfe des Eluiermittels Chloroform-Methanol-konz. Ammoniak 22 : 7 : 1 chromatographiert. Die einheitliche Substanz enthaltenden Fraktionen werden vereinigt, teilweise eingedampft, mit 1-n.Salzsäure angesäuert und mit Äthylacetat extrahiert. Die organischen Phasen werden wie oben erwähnt gewaschen, getrocknet und eingedampft. Das erhaltene gelbe Öl wird mit heissem Hexan bis zur Kristallisation verrieben. Die Kristalle werden abfiltriert und ein zweites Mal mit heissem Hexan verrieben. Man erhält so die gewünschte 5-Benzoyl-6-chlor-2-benzdioxolcarbonsäure als weisses Pulver vom Smp. 146-148°.

Das als Ausgangsmaterial benötigte (4,5-Dihydroxy-2-chlorphenyl)-phenylmethanon kann auf zwei Arten hergestellt werden:

a) Ein Gemisch von 300 ml Schwefelkohlenstoff, 80 g (0,60 Mol) gepulvertes Aluminiumtrichlorid und 28,9 g (0,20 Mol) 2-Chlor-1,2-benzoldiol wird 15 Minuten bei Raumtemperatur, hierauf eine Stunde bei 40° gerührt. Innerhalb 10 Minuten gibt man 30,9 g (0,220 Mol) Benzoylchlorid zu, rührt 15 Minuten bei 40° weiter und erhitzt, um den Schwefelkohlenstoff langsam abzudestillieren. Der trockene Rückstand wird dann innerhalb einer Stunde auf 140° (Badtemperatur) erhitzt und auf dieser Temperatur während 3 1/2 Stunden gehalten. Die braune feste Masse wird abgekühlt, mit 300 ml 3-n.Salzsäure und 200 ml Äthylacetat versetzt und bis zur vollständigen Lösung gerührt. Die organische Phase wird abgetrennt und die wässrige Phase mit Äthylacetat extrahiert. Die vereinigten organischen Phasen werden mit Wasser und gesättigter Natriumchloridlösung gewaschen, über Natriumsulfat getrocknet, mit Kohle behandelt und eingedampft. Der dunkelbraune Rückstand wird in 100 ml 1,2-Dichloräthan gelöst, mit 105 ml n-Hexan versetzt und bis zur Kristallisation verrieben. Die Kristalle werden abgenutscht und aus Dichloräthan/Hexan umkristallisiert. Das als weisse Kristalle erhaltene (2-Chlor-4,5-dihydroxyphenyl)-phenylmethanon (Smp. 130 bis 135°) kann direkt weiterumgesetzt werden.

b) Man kann das vorgenannte Dihydroxyketon auch in zwei Stufen analog Beispiel 9a) und b) erhalten. Das hierzu benötigte (2-Chlor-4,5-dimethoxyphenyl)-phenylmethanon kann durch die Acylierung von 4-Chlor-1,2-dimethoxybenzol mit Benzoylchlorid in Gegenwart von Jod erhalten werden. Man erhält es aber leichter, wenn man ein Äquivalent 4-Chlor-1,2-dimethoxybenzol mit 1,1 Äquivalent (2-Pyridinyl)-benzoat in Trifluoressigsäure 2 Stunden im Bombenrohr auf 150° erhitzt. Nach Aufarbeitung und Demethylierung analog Beispiel 9b) gewinnt man das gewünschte (4,5-Dihydroxy-2-chlorphenyl)-phenylmethanon.

*Beispiel 11*

Tabletten enthaltend 100 mg 5-Benzoyl-6-methyl-2-benzodioxolcarbonsäure können beispielsweise in folgender Zusammensetzung hergestellt werden:

| *Zusammensetzung* | pro Tablette |
|---|---|
| 5-Benzoyl-6-methyl-2-benzodioxolcarbonsäure | 100 mg |
| Milchzucker | 50 mg |
| Weizenstärke | 73 mg |
| Kolloidale Kieselsäure | 13 mg |
| Talk | 12 mg |
| Magnesiumstearat | 2 mg |
| | 250 mg |

*Herstellung*

Der Wirkstoff wird mit Milchzucker, einem Teil der Weizenstärke und mit kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist. Die Masse wird durch ein Sieb von ca. 3 mm Maschenweite getrieben, getrocknet und das trockene Granulat nochmals durch ein Sieb getrieben. Darauf werden die restliche Weizenstärke, Talk und Magnesiumstearat zugemischt. Die erhaltene Mischung wird zu Tabletten von 250 mg mit Bruchkerbe(n) verpresst.

*Beispiel 12*

Um 1000 Kapseln mit je 100 mg Wirkstoffgehalt herzustellen, mischt man 100 g 5-Benzoyl-6-methyl-2-benzodioxolcarbonsäure mit 173,0 g Lactose, befeuchtet die Mischung gleichmässig mit einer wässrigen Lösung von 2,0 g Gelatine und granuliert sie durch ein geeignetes Sieb (z.B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Anstelle von 5-Benzoyl-6-methyl-2-benzodioxolcarbonsäure kann man in den obigen Beispielen auch eine andere Verbindung der allgemeinen Formel I oder ein pharmazeutisch annehmbares Salz einer zur Salzbildung befähigten Verbindung der allgemeinen Formel I, z.B. eine der in den Beispielen 2 bis 8 beschriebenen Verbindungen oder ein pharmazeutisch annehmbares Salz einer solchen, verwenden.

**Patentansprüche für die Vertragsstaaten:**
BE, CH, DE, FR, GB, IT, LI, LU, NL, SE

1. Benzodioxolderivate der allgemeinen Formel I

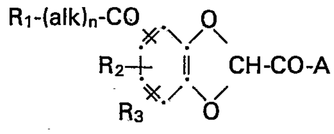

(I)

in welcher $R_1$ einen unsubstituierten oder durch Niederalkyl, Niederalkoxy, Halogen oder Trifluormethyl substituierten aromatischen oder heteroaromatischen Rest, alk einen Alkylen- oder Alkylidenrest mit öchstens 5 Kohlenstoffatomen und n Null oder Eins bedeutet, $R_2$ und $R_3$ unabhängig voneinander Wasserstoff, Niederalkyl, Niederalkoxy oder Halogen bedeuten und A den Rest $-O-R_4$, worin $R_4$ für Wasserstoff oder einen unsubstituierten oder durch Niederalkoxy oder Halogen substituierten aliphatischen oder araliphatischen Kohlenwasserstoffrest steht, oder den Rest

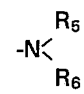

bedeutet, worin $R_5$ und $R_6$ unabhängig voneinander für Wasserstoff oder Niederalkyl, oder unter sich verbunden und zusammen mit dem anliegenden Stickstoffatom für gegebenenfalls niederalkylsubstituiertes Tetra- bis Hexamethylenimino oder 4-Morpholinyl stehen, als Racemate oder optische Antipoden, sowie Salze von Verbindungen der allgemeinen Formel I, in denen A $OR_4$ und darin $R_4$ Wasserstoff bedeutet, mit Basen sowie Säureadditionssalze von Verbindungen der allgemeinen Formel I, deren Rest $R_1$ basischen Charakter aufweist, wobei die mit «nieder» bezeichneten Reste höchstens 7 Kohlenstoffatome aufweisen.

2. Verbindungen der im Anspruch 1 angegebenen allgemeinen Formel I, in denen $R_1$ durch Niederalkyl, Niederalkoxy oder Halogen substituiertes oder unsubstituiertes Phenyl, Thienyl oder Furyl, alk Alkylen oder Alkyliden mit höchstens 3 Kohlenstoffatomen, $R_2$ Niederalkyl oder Halogen, $R_3$ Wasserstoff, Niederalkyl oder Halogen bedeutet und n und A die im Anspruch 1 angegebene Bedeutung haben, als Racemate oder optische Antipoden, sowie Salze von solchen Verbindungen, in denen A $OR_4$ und darin $R_4$ Wasserstoff ist, mit Basen, wobei die mit «nieder» bezeichneten Reste höchstens 7 Kohlenstoffatome aufweisen.

3. Verbindungen der im Anspruch 1 angegebenen allgemeinen Formel I, in denen $R_1$ durch Niederalkyl, Niederalkoxy oder Halogen substituiertes oder unsubstituiertes Phenyl oder Thienyl, alk Methylen, n Null oder Eins, $R_2$ Niederalkyl oder Halogen, $R_3$ Wasserstoff oder Niederalkyl, und A $OR_4$ und darin $R_4$ Wasserstoff oder Niederalkyl bedeutet, als Racemate oder optische Antipoden, sowie die pharmazeutisch annehmbaren Salze von solchen Verbindungen, in denen $R_4$ Wasserstoff ist, mit Basen, wobei die mit «nieder» bezeichneten Reste höchstens 7 Kohlenstoffatome aufweisen.

4. Verbindungen der im Anspruch 1 angegebenen allgemeinen Formel I, in denen $R_1$ durch Niederalkyl, Niederalkoxy oder Halogen substituiertes oder unsubstituiertes Phenyl, alk Methylen, n Null oder Eins, $R_2$ Niederalkyl oder Halogen, $R_3$ Wasserstoff oder Niederalkyl und A $OR_4$ und darin $R_4$ Wasserstoff oder Niederalkyl bedeutet, als Racemate oder optische Antipoden, sowie die pharmazeutisch annehmbaren Salze von solchen Verbindungen, in denen $R_4$ Wasserstoff ist, mit Basen, wobei die mit «nieder» bezeichneten Reste höchstens 7 Kohlenstoffatome aufweisen.

5. Verbindungen der im Anspruch 1 angegebenen allgemeinen Formel I, in denen $R_1$ durch Niederalkyl, Niederalkoxy oder Halogen substituiertes oder unsubstituiertes Phenyl, n Null, $R_2$ Niederalkyl oder Halogen, $R_3$ Wasserstoff und A $OR_4$ und darin $R_4$ Wasserstoff oder Niederalkyl bedeuten, und der Acylrest in 5- oder 6-Stellung gebunden ist, als Racemate oder optische Antipoden, sowie die pharmazeutisch annehmbaren Salze von solchen Verbindungen, in denen $R_4$ Wasserstoff ist, mit Basen, wobei die mit «nieder» bezeichneten Reste höchstens 7 Kohlenstoffatome aufweisen.

6. Die Benzoyl-6-methyl-2-benzodioxolcarbonsäure, ihre optischen Antipoden und ihre Salze mit Basen.

7. Die 5-(4-Fluorbenzoyl)-6-methyl-2-benzodioxolcarbonsäure und ihre Salze mit Basen.

8. Die 5-(4-Methoxybenzoyl)-6-methyl-2-benzodioxolcarbonsäure und ihre Salze mit Basen.

9. Die 5-[(4-Fluorphenyl)-acetyl]-6-methyl-2-benzodioxolcarbonsäure und ihre Salze mit Basen.

10. Die 5-Methyl-6-[(2-thienyl)-carbonyl]-2-benzodioxolcarbonsäure und ihre Salze mit Basen.

11. Die 5-Benzoyl-6-fluor-2-benzodioxolcarbonsäure und ihre Salze mit Basen.

12. Die 5-Benzoyl-6-chlor-2-benzodioxolcarbonsäure und ihre Salze mit Basen.

13. Pharmazeutische Präparate, enthaltend eine Verbindung gemäss einem der Ansprüche 1 bis 12 oder ein pharmazeutisch annehmbares Salz einer zur Salzbildung befähigten Verbindung gemäss Anspruch 1 oder einer Verbindung gemäss einem der Ansprüche 2 bis 12.

14. Verbindungen gemäss einem der Ansprüche 1 bis 12 und pharmazeutisch annehmbare Salze von zur Salzbildung befähigten Verbindungen gemäss Anspruch 1 oder von Verbindungen gemäss einem der Ansprüche 2 bis 12 zur Anwendung in einem Verfahren zur prophylaktischen und/oder therapeutischen Behandlung des tierischen oder menschlichen Körpers.

15. Verbindungen gemäss einem der Ansprüche 1 bis 12 und pharmazeutisch annehmbare Salze von zur Salzbildung befähigten Verbindungen gemäss Anspruch 1 oder einer Verbindung gemäss einem der Ansprüche 2 bis 12 als Diuretika mit urikosurischer Zusatzwirkung.

16. Verwendung von Verbindungen gemäss einem der Ansprüche 1 bis 12 oder von pharmazeutisch annehmbaren Salzen von zur Salzbildung befähigten Verbindungen gemäss Anspruch 1 oder einer Verbindung gemäss einem der Ansprüche 2 bis 12 zur Herstellung von Arzneimitteln auf nichtchemischen Wege.

17. Verfahren zur Herstellung von Benzodioxolderivaten der im Anspruch 1 angegebenen allgemeinen Formel I, in welcher $R_1$, $R_2$, $R_3$, alk, n und A die dort definierte Bedeutung haben, und von Salzen derselben, dadurch gekennzeichnet, dass man

a) eine Verbindung der allgemeinen Formel II

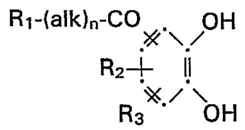 (II)

in welcher $R_1$, alk, n, $R_2$ und $R_3$ die im Anspruch 1 angegebene Bedeutung haben, oder ein Salz davon mit einer Verbindung der allgemeinen Formel III

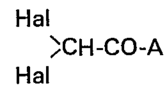 (III)

in welcher Hal Halogen bedeutet und A die im Anspruch 1 angegebene Bedeutung hat, oder einem Salz einer solchen Verbindung, in der A $OR_4$ und darin $R_4$ Wasserstoff bedeutet, umsetzt, oder

b) in einer Verbindung der allgemeinen Formel IV

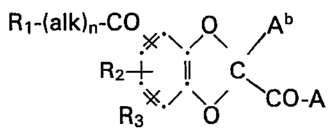 (IV)

in welcher $A^b$ Carboxy, Niederalkoxycarbonyl oder Acetyl bedeutet, und $R_1$, alk, n, $R_2$, $R_3$ und A die im Anspruch 1 angegebene Bedeutung haben, den Rest $A^b$ durch Wasserstoff ersetzt, oder

c) zur Herstellung einer Verbindung der allgemeinen Formel I, in welcher A die im Anspruch 1 angegebene Bedeutung hat mit Ausnahme eines Restes $OR_4$, in welchem $R_4$ Wasserstoff bedeutet, und $R_1$, alk, n, $R_2$ und $R_3$ die im Anspruch angegebene Bedeutung haben, eine Verbindung der allgemeinen Formel V

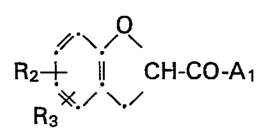 (V)

mit einem Anhydrid einer Verbindung der allgemeinen Formel VI,

$$R_1\text{-(alk)}_n\text{-CO-OH} \qquad (VI)$$

worin $A_1$ die im Anspruch 1 für A angegebene Bedeutung hat mit Ausnahme eines Restes $OR_4$, in welchem $R_4$ Wasserstoff bedeutet, und $R_2$ und $R_3$ bzw. $R_1$, alk und n die im Anspruch 1 angegebene Bedeutung haben, umsetzt, oder

d) zur Herstellung einer Verbindung der allgemeinen Formel I, in welcher A $OR_4$ und darin $R_4$ Wasserstoff bedeutet und $R_1$, alk, n, $R_2$ und $R_3$ die im Anspruch 1 angegebene Bedeutung haben, oder eines Salzes dieser Verbindung, in einer Verbindung der allgemeinen Formel VII

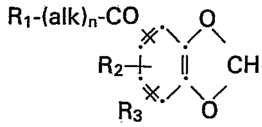

(VII)

in welcher $A^d$ eine in die Carboxygruppe überführbare Gruppe bedeutet und $R_1$, alk, n, $R_2$ und $R_3$ die im Anspruch 1 angegebene Bedeutung haben, die Gruppe $A^d$ in die Carboxygruppe in freier oder Salzform überführt, oder

e) zur Herstellung einer Verbindung der allgemeinen Formel I, in welcher A die im Anspruch 1 angegebene Bedeutung hat mit Ausnahme eines Restes $OR_4$, in welchem $R_4$ Wasserstoff bedeutet, und $R_1$, alk, n, $R_2$ und $R_3$ die im Anspruch 1 angegebene Bedeutung haben, in einer Verbindung der allgemeinen Formel VIII

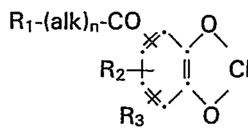

(VIII)

in welcher $A^e$ einen in einen Rest $CO-A_1$, in welchem $A_1$ die im Anspruch 1 für A angegebene Bedeutung hat mit Ausnahme eines Restes $OR_4$, in welchem $R_4$ Wasserstoff bedeutet, überführbaren Rest bedeutet, und $R_1$, alk, n, $R_2$ und $R_3$ die im Anspruch 1 angegebene Bedeutung haben, den Rest $A^e$ in den Rest $-CO-A_1$ überführt, und gewünschtenfalls eine erhaltene Verbindung der allgemeinen Formel I in an sich bekannter Weise in eine andere Verbindung der allgemeinen Formel I, und/oder eine erhaltene Verbindung der allgemeinen Formel I, in welcher A $OR_4$ und darin $R_4$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

18. Die nach den Verfahren des Anspruchs 17 erhältlichen Verbindungen der im Anspruch 1 angegebenen allgemeinen Formel I, in welcher $R_1$, alk, n, $R_2$, $R_3$ und A die dort definierte Bedeutung haben, und Salze von zur Salzbildung befähigten, nach diesen Verfahren erhältlichen Verbindungen der allgemeinen Formel I.

**Patentansprüche für den Vertragsstaat:** AT

1. Verfahren zur Herstellung von Benzodioxolderivaten der allgemeinen Formel I

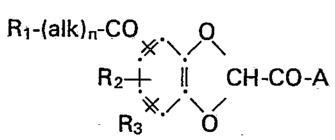

(I)

in welcher $R_1$ einen unsubstituierten oder durch Niederalkyl, Niederalkoxy, Halogen oder Trifluormethyl substituierten aromatischen oder heteroaromatischen Rest, alk einen Alkylen- oder Alkylidenrest mit höchstens 5 Kohlenstoffatomen und n Null oder Eins bedeutet, $R_2$ und $R_3$ unabhängig voneinander Wasserstoff, Niederalkyl, Niederalkoxy oder Halogen bedeuten und A den Rest $-O-R_4$, worin $R_4$ für Wasserstoff oder einen unsubstituierten oder durch Niederalkoxy oder Halogen substituierten aliphatischen oder araliphatischen Kohlenwasserstoffrest steht, oder den Rest

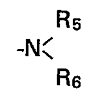

bedeutet, worin $R_5$ und $R_6$ unabhängig voneinander für Wasserstoff oder Niederalkyl, oder unter sich verbunden und zusammen mit dem anliegenden Stickstoffatom für gegebenenfalls niederalkylsubstituiertes Tetra- bis Hexamethylenimino oder 4-Morpholinyl stehen, und von Salzen derselben, wobei die mit «nieder» bezeichneten Reste höchstens 7 Kohlenstoffatome aufweisen, dadurch gekennzeichnet, dass man

a) eine Verbindung der allgemeinen Formel II

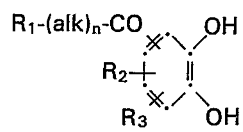

(II)

in welcher $R_1$, alk, n, $R_2$ und $R_3$ die unter Formel I angegebene Bedeutung haben, oder ein Salz davon mit einer Verbindung der allgemeinen Formel III

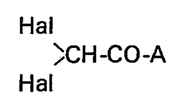

(III)

in welcher Hal Halogen bedeutet und A die unter Formel I angegebene Bedeutung hat, oder einem Salz einer solchen Verbindung, in der A $OR_4$ und darin $R_4$ Wasserstoff bedeutet, umsetzt, oder

b) in einer Verbindung der allgemeinen Formel IV

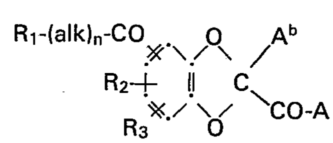

(IV)

in welcher $A^b$ Carboxy, Niederalkoxycarbonyl oder Acetyl bedeutet, und $R_1$, alk, n, $R_2$, $R_3$ und A die unter Formel I angegebene Bedeutung haben, den Rest $A^b$ durch Wasserstoff ersetzt, oder

c) zur Herstellung einer Verbindung der allgemeinen Formel I, in welcher A die unter Formel I angegebene Bedeutung hat mit Ausnahme eines Restes $OR_4$, in welchem $R_4$ Wasserstoff bedeutet, und $R_1$, alk, n, $R_2$ und $R_3$ die unter Formel I angegebene Bedeutung haben, eine Verbindung der allgemeinen Formel V

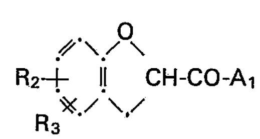

(V)

mit einem Anhydrid einer Verbindung der allgemeinen Formel VI,

$$R_1\text{-(alk)}_n\text{-CO-OH} \qquad \text{(VI)}$$

worin $A_1$ die unter Formel I für A angegebene Bedeutung hat mit Ausnahme eines Restes $OR_4$, in welchem $R_4$ Wasserstoff bedeutet, und $R_2$ und $R_3$ bzw. $R_1$, alk und n die unter Formel I angegebene Bedeutung haben, umsetzt, oder

d) zur Herstellung einer Verbindung der allgemeinen Formel I, in welcher A $OR_4$ und darin $R_4$ Wasserstoff bedeutet und $R_1$, alk, n, $R_2$ und $R_3$ die unter Formel I angegebene Bedeutung haben, oder eines Salzes dieser Verbindung, in einer Verbindung der allgemeinen Formel VII

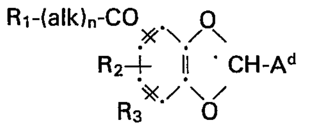

(VII)

in welcher $A^d$ eine in die Carboxygruppe überführbare Gruppe bedeutet und $R_1$, alk, n, $R_2$ und $R_3$ die unter Formel I angegebene Bedeutung haben, die Gruppe $A^d$ in die Carboxygruppe in freier oder Salzform überführt, oder

e) zur Herstellung einer Verbindung der allgemeinen Formel I, in welcher A die unter Formel I angegebene Bedeutung hat mit Ausnahme eines Restes $OR_4$, in welchem $R_4$ Wasserstoff bedeutet, und $R_1$, alk, n, $R_2$ und $R_3$ die unter Formel I angegebene Bedeutung haben, in einer Verbindung der allgemeinen Formel VIII

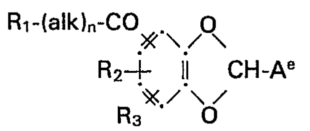

(VIII)

in welcher $A^e$ einen in einen Rest $CO\text{-}A_1$, in welchem $A_1$ die unter Formel I für A angegebene Bedeutung hat mit Ausnahme eines Restes $OR_4$, in welchem $R_4$ Wasserstoff bedeutet, überführbaren Rest bedeutet, und $R_1$, alk, n, $R_2$ und $R_3$ die unter Formel I angegebene Bedeutung haben, den Rest $A^e$ in den Rest $-CO\text{-}A_1$ überführt, und gewünschtenfalls eine erhaltene Verbindung der allgemeinen Formel I in an sich bekannter Weise in eine andere Verbindung der allgemeinen Formel I, und/oder eine erhaltene Verbindung der allgemeinen Formel I, in welcher A $OR_4$ und darin $R_4$ Wasserstoff bedeutet, in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

2. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl, Thienyl oder Furyl, alk Alkylen oder Alkyliden mit höchstens 3 Kohlenstoffatomen, $R_2$ Niederalkyl oder Halogen, $R_3$ Wasserstoff, Niederalkyl oder Halogen bedeutet und n und A die im Anspruch 1 angegebene Bedeutung haben, als Racemate oder optische Antipoden, sowie Salze von solchen Verbindungen, in denen A $OR_4$ und darin $R_4$ Wasserstoff ist, mit Basen herstellt, wobei die mit «nieder» bezeichneten Reste höchstens 7 Kohlenstoffatome aufweisen.

3. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl oder Thienyl, alk Methylen, n Null oder Eins, $R_2$ Niederalkyl oder Halogen, $R_3$ Wasserstoff oder Niederalkyl, und A $OR_4$ und darin $R_4$ Wasserstoff oder Niederalkyl bedeutet, als Racemate oder optische Antipoden, sowie die pharmazeutisch annehmbaren Salze von solchen Verbindungen, in denen $R_4$ Wasserstoff ist, mit Basen herstellt, wobei die mit «nieder» bezeichneten Reste höchstens 7 Kohlenstoffatome aufweisen.

4. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl, alk Methylen, n Null oder Eins, $R_2$ Niederalkyl oder Halogen, $R_3$ Wasserstoff oder Niederalkyl und A $OR_4$ und darin $R_4$ Wasserstoff oder Niederalkyl bedeutet, als Racemate oder optische Antipoden, sowie die pharmazeutisch annehmbaren Salze von solchen Verbindungen, in denen $R_4$ Wasserstoff ist, mit Basen herstellt, wobei die mit «nieder» bezeichneten Reste höchstens 7 Kohlenstoffatome aufweisen.

5. Verfahren nach Anspruch 1, dadurch gekennzeichnet, dass man Verbindungen der im Anspruch 1 angegebenen allgemeinen Formel I, in denen $R_1$ durch Niederalkyl, Niederalkoxy oder Halogen substituiertes oder unsubstituiertes Phenyl, n Null, $R_2$ Niederalkyl oder Halogen, $R_3$ Wasserstoff und A $OR_4$ und darin $R_4$ Wasserstoff oder Niederalkyl bedeuten, und der Acylrest in 5- oder 6-Stellung gebunden ist, als Racemate oder optische Antipoden, sowie die pharmazeutisch annehmbaren Salze von solchen Verbindungen, in denen $R_4$ Wasserstoff ist, mit Basen herstellt, wobei die mit «nieder» bezeichneten Reste höchstens 7 Kohlenstoffatome aufweisen.

6. Verfahren nach Anspruch 1, dadurch gekennzeichnet, dass man 5-Benzoyl-6-methyl-2-benzodioxolcarbonsäure, ihre optischen Antipoden und ihre Salze mit Basen herstellt.

7. Verfahren nach Anspruch 1, dadurch gekennzeichnet, dass man 5-(4-Fluorbenzoyl)-6-methyl-2--benzodioxolcarbonsäure und ihre Salze mit Basen herstellt.

8. Verfahren nach Anspruch 1, dadurch gekennzeichnet, dass man 5-(4-Methoxybenzoyl)-6-methyl-2-benzodioxolcarbonsäure und ihre Salze mit Basen herstellt.

9. Verfahren nach Anspruch 1, dadurch gekennzeichnet, dass man 5-[(4-Fluorphenyl)-acetyl]-6-methyl-2-benzodioxolcarbonsäure und ihre Salze mit Basen herstellt.

10. Verfahren nach Anspruch 1, dadurch gekennzeichnet, dass man 5-Methyl-6-[(2-thienyl)-carbonyl]-2-benzodioxolcarbonsäure und ihre Salze mit Basen herstellt.

11. Verfahren nach Anspruch 1, dadurch gekennzeichnet, dass man 5-Benzoyl-6-fluor-2-benzodioxolcarbonsäure und ihre Salze mit Basen herstellt.

12. Verfahren nach Anspruch 1, dadurch gekennzeichnet, dass man 5-Benzoyl-6-chlor-2-benzodioxolcarbonsäure und ihre Salze mit Basen herstellt.

**Claims for the Contracting States:**
DE, GB, FR, CH, LI, IT, NL, BE, SE, LU

1. A benzodioxole derivative of the general formula I

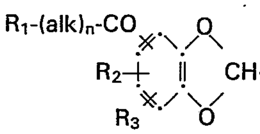
(I)

wherein $R_1$ is an aromatic or heteroaromatic radical which is unsubstituted or substituted by lower alkyl, lower alkoxy, halogen or trifluoromethyl, alk is an alkylene or alkylidene radical containing not more than 5 carbon atoms, n is 0 or 1, $R_2$ and $R_3$ are each independently hydrogen, lower alkyl, lower alkoxy or halogen, and A is the radical $-O-R_4$, in which $R_4$ is hydrogen or an aliphatic or araliphatic hydrocarbon radical which is unsubstituted or substituted by lower alkoxy or halogen, or A is the radical

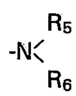

wherein $R_5$ and $R_6$ are each independently hydrogen or lower alkyl, or $R_5$ and $R_6$ are bonded to each other and, together with the adjacent nitrogen atom, are unsubstituted or lower alkyl-substituted tetra- to hexamethylenimino or 4-morpholinyl, in the form of a racemate or optical antipode, or a salt of a compound of the general formula I, wherein A is $OR_4$, in which $R_4$ is hydrogen, with a base, or an acid addition salt of a compound of formula I, wherein the radical $R_1$ has basic character, with the radicals qualified by the term «lower» containing not more than 7 carbon atoms.

2. A compound of the general formula I according to claim 1, wherein $R_1$ is phenyl, thienyl or furyl, each unsubstituted or substituted by lower alkyl, lower alkoxy or halogen, alk is alkylene or alkylidene containing not more than 3 carbon atoms, $R_2$ is lower alkyl or halogen, $R_3$ is hydrogen, lower alkyl or halogen, and n and A are as defined in claim 1, in the form of a racemate or optical antipode, or a salt of such a compound, wherein A is $OR_4$, in which $R_4$ is hydrogen, with a base, with the radicals qualified by the term «lower» containing not more than 7 carbon atoms.

3. A compound of the general formula I according to claim 1, wherein $R_1$ is phenyl or thienyl, each unsubstituted or substituted by lower alkyl, lower alkoxy or halogen, alk is methylene, n is 0 or 1, $R_2$ is lower alkyl or halogen, $R_3$ is hydrogen or lower alkyl, and A is $OR_4$, in which $R_4$ is hydrogen or lower alkyl, in the form of a racemate or optical antipode, or a pharmaceutically acceptable salt of such a compound, in which $R_4$ is hydrogen, with a base, with the radicals qualified by the term «lower» containing not more than 7 carbon atoms.

4. A compound of the general formula I according to claim 1, wherein $R_1$ is phenyl which is unsubstituted or substituted by lower alkyl, lower alkoxy or halogen, alk is methylene, n is 0 or 1, $R_2$ is lower alkyl or halogen, $R_3$ is hydrogen or lower alkyl, and A is $OR_4$, in which $R_4$ is hydrogen or lower alkyl, in the form of a racemate or an optical antipode, or a pharmaceutically acceptable salt of such a compound, in which $R_4$ is hydrogen, with a base, with the radicals qualified by the term «lower» containing not more than 7 carbon atoms.

5. A compound of the general formula I according to claim 1, wherein $R_1$ is phenyl which is unsubstituted or substituted by lower alkyl, lower alkoxy or halogen, n is 0, $R_2$ is lower alkyl or halogen, $R_3$ is hydrogen, and A is $OR_4$, in which $R_4$ is hydrogen or lower alkyl, and the acyl radical is bonded in the 5- or 6-position, in the form of a racemate or an optical antipode, or a pharmaceutically acceptable salt of such a compound, in which $R_4$ is hydrogen, with a base, with the radicals qualified by the term «lower» containing not more than 7 carbon atoms.

6. Benzoyl-6-methyl-2-benzodioxolecarboxylic acid, or an optical antipode thereof, or a salt thereof with a base.

7. 5-(4-Fluorobenzoyl)-6-methyl-2-benzodioxolecarboxylic acid, or a salt thereof with a base.

8. 5-(4-Methoxybenzoyl)-6-methyl-2-benzodioxolecarboxylic acid, or a salt thereof with a base.

9. 5-[(4-Fluorophenyl)acetyl]-6-methyl-2-benzodioxolecarboxylic acid, or a salt thereof with a base.

10. 5-Methyl-6-[(2-thienyl)carbonyl]-2-benzodioxolecarboxylicacid, or a salt thereof with a base.

11. 5-Benzoyl-6-fluoro-2-benzodioxolecarboxylic acid, or a salt thereof with a base.

12. 5-Benzoyl-6-chloro-2-benzodioxolecarboxylic acid, or a salt thereof with a base.

13. A pharmaceutical composition containing a compound according to any one of claims 1 to 12, or a pharmaceutically acceptable salt of a compound capable of salt-formation according to claim 1 or of a compound according to any one of claims 2 to 12.

14. A compound according to any one of claims 1 to 12, or a pharmaceutically acceptable salt of a compound capable of salt-formation according to claim 1 or of a compound according to any one of claims 2 to 12, for use in a method of treating the animal or human body prophylactically or therapeutically.

15. Use of a compound according to any one of claims 1 to 12, or a pharmaceutically acceptable salt of a compound capable of salt-formation according to claim 1 or of a compound according to any one of claims 2 to 12, as diuretic with additional uricosuric action.

16. Use of a compound according to any one of claims 1 to 12, or of a pharmaceutically acceptable salt of a compound capable of salt formation according to claim 1 or of a compound according to

any one of claims 2 to 12, for the preparation of a pharmaceutical composition by non-chemical means.

17. A process for the preparation of a benzodioxole derivative of the general formula I according to claim 1, in which formula $R_1$, $R_2$, $R_3$, alk, n and A are as defined in claim 1, or of a salt thereof, which process comprises

a) reacting a compound of the general formula II

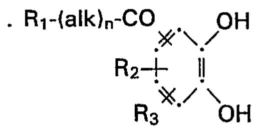

(II)

wherein $R_1$, alk, n, $R_2$ and $R_3$ are as defined in claim 1, or a salt thereof, with a compound of the general formula III

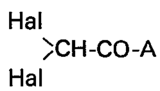

(III)

wherein Hal is halogen and A is as defined in claim 1, or with a salt of such a compound, wherein A is $OR_4$, in which $R_4$ is hydrogen; or

b) in a compound of the general formula IV

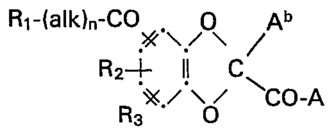

(IV)

wherein $A^b$ is carboxy, lower alkoxycarbonyl or acetyl, and $R_1$, alk, n, $R_2$, $R_3$ and A are as defined in claim 1, replacing the radical $A^b$ by hydrogen; or

c) to prepare a compound of the general formula I, wherein A is as defined in claim 1, with the exception of a radical $OR_4$, in which $R_4$ is hydrogen, and $R_1$, alk, n, $R_2$ and $R_3$ are as defined in claim 1, reacting a compound of the general formula V

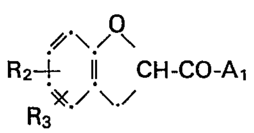

(V)

with an anhydride of a compound of the general formula VI

$R_1$-(alk)$_n$-CO-OH (VI)

wherein $A_1$ has the same meaning as A as defined in claim 1, with the exception of a radical $OR_4$, in which $R_4$ is hydrogen, and $R_2$ and $R_3$ and $R_1$, alk and n are as defined in claim 1; or

d) to prepare a compound of the general formula I, wherein A is $OR_4$, in which $R_4$ is hydrogen, and $R_1$, alk, n, $R_2$ and $R_3$ are as defined in claim 1, or a salt of such a compound, in a compound of the general formula VII

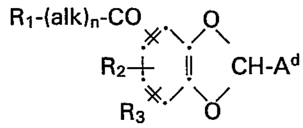

(VII)

wherein $A^d$ is a group which can be converted into a carboxyl group, and $R_1$, alk, n, $R_2$ and $R_3$ are as defined in claim 1, converting the group $A^d$ into the carboxyl group in free or salt form; or

e) to prepare a compound of the general formula I, wherein A is as defined in claim 1, with the exception of a radical $OR_4$, in which $R_4$ is hydrogen, and $R_1$, alk, n, $R_2$ and $R_3$ are as defined in claim 1, in a compound of the general formula VIII

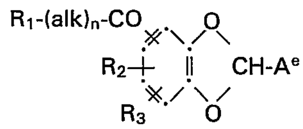

(VIII)

wherein $A^e$ is a radical which can be converted into a radical -CO-$A_1$, wherein $A_1$ has the same meaning as A as defined in claim 1, with the exception of a radical $OR_4$, in which $R_4$ is hydrogen, and $R_1$, alk, n, $R_2$ and $R_3$ are as defined in claim 1, converting the radical $A^e$ into the radical -CO-$A_1$, and, if desired, converting a resultant compound of the general formula I into another compound of the general formula I in a manner known per se, and/or converting a resultant compound of the general formula I, wherein A is $OR_4$, in which $R_4$ is hydrogen, into a salt with a base, or liberating such a compound from a resultant salt, or converting a resultant compound of the general formula I of basic character into an acid addition salt, or liberating such a compound from a resultant salt.

18. A compound of the general formula I according to claim 1, wherein $R_1$, alk, n, $R_2$, $R_3$ and A are as defined in claim 1, which compound is obtainable by a process according to claim 17, or a salt of a compound of the general formula I capable of salt-formation and obtainable by a process according to claim 17.

**Claims for the Contracting State:** AT

1. A process for the preparation of a benzodioxole derivative of the general formula I

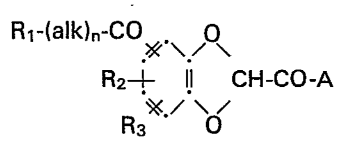

(I)

wherein $R_1$ is an aromatic or heteroaromatic radical which is unsubstituted or substituted by lower alkyl, lower alkoxy, halogen or trifluoromethyl, alk is an alkylene or alkylidene radical containing not more than 5 carbon atoms, n is 0 or 1, $R_2$ and $R_3$ are each independently hydrogen, lower alkyl, lower alkoxy or halogen, and A is the radical -O-$R_4$, in which $R_4$ is hydrogen or an aliphatic or araliphatic hydrocarbon

radical which is unsubstituted or substituted by lower alkoxy or halogen, or A is the radical

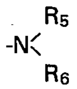

wherein $R_5$ and $R_6$ are each independently hydrogen or lower alkyl, or $R_5$ and $R_6$ are bonded to each other and, together with the adjacent nitrogen atom, are unsubstituted or lower alkyl-substituted tetra- to hexamethylenimino or 4-morpholinyl, or of a salt thereof, with the radicals qualified by the term «lower» containing not more than 7 carbon atoms, which process comprises

a) reacting a compound of the general formula II

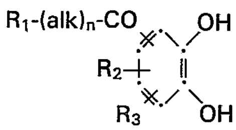 (II)

wherein $R_1$, alk, n, $R_2$ and $R_3$ are as defined in claim 1, or a salt thereof, with a compound of the general formula III

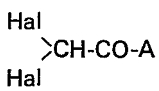 (III)

wherein Hal is halogen and A is as defined in claim 1, or with a salt of such a compound, wherein A is $OR_4$, in which $R_4$ is hydrogen; or

b) in a compound of the general formula IV

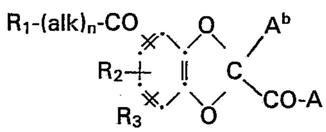 (IV)

wherein $A^b$ is carboxy, lower alkoxycarbonyl or acetyl, and $R_1$, alk, n, $R_2$, $R_3$ and A are as defined in claim 1, replacing the radical $A^b$ by hydrogen; or

c) to prepare a compound of the general formula I, wherein A is as defined in claim 1, with the exception of a radical $OR_4$, in which $R_4$ is hydrogen, and $R_1$, alk, n, $R_2$ and $R_3$ are as defined in claim 1, reacting a compound of the general formula V

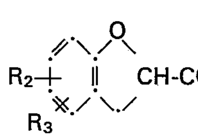 (V)

with an anhydride of a compound of the general formula VI

$R_1$-$(alk)_n$-CO-OH (VI)

wherein $A_1$ has the same meaning as A as defined in claim 1, with the exception of a radical $OR_4$, in which $R_4$ is hydrogen, and $R_2$ and $R_3$ and $R_1$, alk and n are as defined in claim 1; or

d) to prepare a compound of the general formula I, wherein A is $OR_4$, in which $R_4$ is hydrogen, and $R_1$, alk, n, $R_2$ and $R_3$ are defined in claim 1, or a salt of such a compound, in a compound of the general formula VII

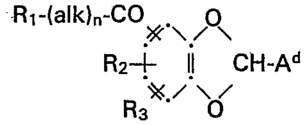 (VII)

wherein $A^d$ is a group which can be converted into a carboxyl group, and $R_1$, alk, n, $R_2$ and $R_3$ are as defined in claim 1, converting the group $A^d$ into the carboxyl group in free or salt form; or

e) to prepare a compound of the general formula I, wherein A is as defined in claim 1, with the exception of a radical $OR_4$, in which $R_4$ is hydrogen, and $R_1$, alk, n, $R_2$ and $R_3$ are as defined in claim 1, in a compound of the general formula VIII

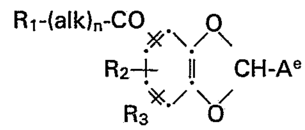 (VIII)

wherein $A^e$ is a radical which can be converted into a radical -CO-$A_1$, wherein $A_1$ has the same meaning as A as defined in claim 1, with the exception of a radical $OR_4$, in which $R_4$ is hydrogen, and $R_1$, alk, n, $R_2$ and $R_3$ are as defined in claim 1, converting the radical $A^e$ into the radical -CO-$A_1$, and, if desired, converting a resultant compound of the general formula I into another compound of the general formula I in a manner known per se, and/or converting a resultant compound of the general formula I, wherein A is $OR_4$, in which $R_4$ is hydrogen, into a salt with a base, or liberating such a compound from a resultant salt, or converting a resultant compound of the general formula I of basic character into an acid addition salt, or liberating such a compound from a resultant salt.

2. A process according to claim 1, which comprises preparing a compound of the general formula I according to claim 1, wherein $R_1$ is phenyl, thienyl or furyl, each unsubstituted by lower alkyl, lower alkoxy or halogen, alk is alkylene or alkylidene containing not more than 3 carbon atoms, $R_2$ is lower alkyl or halogen, $R_3$ is hydrogen, lower alkyl or halogen, and n and A are as defined in claim 1, in the form of a racemate or optical antipode, or a salt of such a compound, wherein A is $OR_4$, in which $R_4$ is hydrogen, with a base, with the radicals qualified by the term «lower» containing not more than 7 carbon atoms.

3. A process according to claim I, which comprises preparing a compound of the general formula I according to claim 1, wherein $R_1$ is phenyl or thienyl, each unsubstituted or substituted by lower alkyl, lower alkoxy or halogen, alk is methylene, n is 0 or 1, $R_2$ is lower alkyl or halogen, $R_3$ is hydrogen or lower alkyl, and A is $OR_4$, in which $R_4$ is hydrogen or lower alkyl, in the form of a racemate or optical antipode, or a pharmaceutically acceptable salt of such a compound, in which $R_4$ is hydrogen, with a base, with the radicals qualified by the term «lower» containing not more than 7 carbon atoms.

4. A process according to claim 1, which comprises preparing a compound of the general formula I according to claim 1, wherein $R_1$ is phenyl which is unsubstituted or substituted by lower alkyl, lower alkoxy or halogen, alk is methylene, n is 0 or 1, $R_2$ is lower alkyl or halogen, $R_3$ is hydrogen or lower alkyl, amd A is OR, in which $R_4$ is hydrogen or lower alkyl, in the form of a racemate or an optical antipode, or a pharmaceutically acceptable salt of such a compound, in which $R_4$ is hydrogen, with a base, with the radicals qualified by the term «lower» containing not more than 7 carbon atoms.

5. A process according to claim 1, which comprises preparing a compound of the general formula I according to claim 1, wherein $R_1$ is phenyl which is unsubstituted or substituted by lower alkyl, lower alkoxy or halogen, n is 0, $R_2$ is lower alkyl or halogen, $R_3$ is hydrogen, and A is $OR_4$, in which $R_4$ is hydrogen or lower alkyl, and the acyl radical is bonded in the 5- or 6-position, in the form of a racemate or an optical antipode, or a pharmaceutically acceptable salt of such a compound, in which $R_4$ is hydrogen, with the radicals qualified by the term «lower» containing not more than 7 carbon atoms.

6. A process according to claim 1, which comprises preparing benzoyl-6-methyl-2-benzodioxolecarboxylic acid, or an optical antipode thereof, or a salt thereof with a base.

7. A process according to claim 1, which comprises preparing 5-(4-fluorobenzoyl)-6-methyl-2--benzodioxolecarboxylic acid, or a salt thereof with a base.

8. A process according to claim 1, which comprises preparing 5-(4-methoxybenzoyl)-6-methyl-2--benzodioxolecarboxylic acid, or a salt thereof with a base.

9. A process according to claim 1, which comprises preparing 5-[(4-fluorophenyl)acetyl]-6-methyl-2-benzodioxolecarboxylic acid, or a salt thereof with a base.

10. A process according to claim 1, which comprises preparing 5-methyl-6-[(2-thienyl)carbonyl]-2--benzodioxolecarboxylic acid, or a salt thereof with a base.

11. A process according to claim 1, which comprises preparing 5-benzoyl-6-fluoro-2-benzodioxolecarboxylic acid, or a salt thereof with a base.

12. A process according to claim 1, which comprises preparing 5-benzoyl-6-chloro-2-benzodioxolecarboxylic acid, or a salt thereof with a base.

**Revendications pour les Etats contractants:**
DE, GB, FR, CH, LI, IT, NL, BE, SE, LU

1. Dérivés du benzodioxole de formule générale I

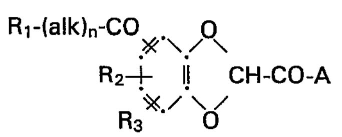

(I)

dans laquelle $R_1$ représente un reste aromatique ou hétéroaromatique non substitué ou substitué par des groupes alkyle inférieurs, alcoxy inférieurs, des halogènes ou des groupes trifluorométhyle, alk représente un groupe alkylène ou alkylidène à 5 atomes de carbone au maximum et n est égal à 0 ou 1, $R_2$ et $R_3$ représentent chacun indépendamment l'un de l'autre, l'hydrogène, un groupe alkyle inférieur, alcoxy inférieur ou un halogène et A représente le reste $-O-R_4$ dans lequel $R_4$ représente l'hydrogène ou un reste hydrocarboné aliphatique ou araliphatique non substitué ou substitué par des groupes alcoxy inférieurs ou des atomes d'halogènes, ou le reste:

$$-N\begin{matrix}R_5\\R_6\end{matrix}$$

dans lequel $R_5$ et $R_6$ représentent chacun, indépendamment l'un de l'autre, l'hydrogène ou un groupe alkyle inférieur, ou bien forment ensemble et avec l'atome d'azote voisin un groupe tétra- à hexa-méthylène-imino ou 4-morpholinyle portant éventuellement des substituants alkyle inférieurs, à l'état de racémates ou d'antipodes optiques, ainsi que les sels des composés de formule générale I dans laquelle A représente $OR_4$ et $R_4$ représente l'hydrogène, et de bases, et les sels des composés de formule générale I dont le reste $R_1$ présente un caractère basique, formés par addition avec des acides, l'expression «inférieur» s'appliquant à des restes qui contiennent au maximum 7 atomes de carbone.

2. Composés de formule générale I de la revendication 1, dans lesquels $R_1$ représente un groupe phényle, thiényle ou furyle substitué par des groupes alkyle inférieurs, alcoxy inférieurs ou des halogènes, ou non substitué, alk représente un groupe alkylène ou alkylidène à 3 atomes de carbone au maximum, $R_2$ représente un groupe alkyle inférieur ou un halogène, $R_3$ représente l'hydrogène, un groupe alkyle inférieur ou un halogène et n et A ont les significations indiquées dans la revendication 1, à l'état de racémates ou antipodes optiques, ainsi que les sels de ces composés dans lesquels A représente $OR_4$ et $R_4$ représente l'hydrogène, et de bases, l'expression «inférieur» s'appliquant à des groupes qui contiennent au maximum 7 atomes de carbone.

3. Composés de formule générale I de la revendication 1, dans lesquels $R_1$ représente un groupe phényle ou thiényle substitué par des groupes alkyle inférieurs, alcoxy inférieurs ou des halogènes, ou non substitué, alk représente un groupe méthylène, n est égal à 0 ou 1, $R_2$ représente un groupe alkyle inférieur ou un halogène, $R_3$ représente l'hydrogène ou un groupe alkyle inférieur et A représente $OR_4$ dans lequel $R_4$ représente l'hydrogène ou un groupe alkyle inférieur, à l'état de racémates et d'antipodes optiques, ainsi que les sels acceptables pour l'usage pharmaceutique de ces composés dans lesquels $R_4$ représente l'hydrogène, et de bases, l'expression «inférieur» s'appliquant à des groupes qui contiennent au maximum 7 atomes de carbone.

4. Composés de formule générale I de la revendication 1, dans lesquels $R_1$ représente un groupe phényle substitué par des groupes alkyle inférieurs, alcoxy inférieurs ou des halogènes ou non substitué, alk représente un groupe méthylène, n est égal à 0 ou 1, $R_2$ représente un groupe alkyle inférieur ou un halogène, $R_3$ représente l'hydrogène ou un groupe

alkyle inférieur et A représente $OR_4$ dans lequel $R_4$ représente l'hydrogène ou un groupe alkyle inférieur, à l'état de racémates ou d'antipodes optiques, ainsi que les sels acceptables pour l'usage pharmaceutique de ces composés dans lesquels $R_4$ représente l'hydrogène, et de bases, l'expression «inférieur» s'appliquant à des groupes qui contiennent au maximum 7 atomes de carbone.

5. Composés de formule générale I de la revendication 1, dans lesquels $R_1$ représente un groupe phényle substitué par des groupes alkyle inférieurs, alcoxy inférieurs ou des halogènes ou non substitué, n est égal à 0, $R_2$ représente un groupe alkyle inférieur ou un halogène, $R_3$ représente l'hydrogène et A représente $OR_4$ dans lequel $R_4$ représente l'hydrogène ou un groupe alkyle inférieur, et le radical acyle est fixé en position 5 ou 6, à l'état de racémates ou d'antipodes optiques, ainsi que les sels acceptables pour l'usage pharmaceutique de ces composés dans lesquels $R_4$ représente l'hydrogène, et de bases, l'expression «inférieur» s'appliquant à des groupes qui contiennent au maximum 7 atomes de carbone.

6. L'acide benzoyl-6-méthyl-2-benzodioxole-carboxylique, ses antipodes optiques et ses sels de bases.

7. L'acide 5-(4-fluorobenzoyl)-6-méthyl-2-benzodioxole-carboxylique et ses sels de bases.

8. L'acide 5-(4-méthoxybenzoyl)-6-méthyl-2--benzodioxole-carboxylique et ses sels de bases.

9. L'acide 5-[(4-fluorophényl)-acétyl]-6-méthyl--2-benzodioxole-carboxylique et ses sels de bases.

10. L'acide 5-méthyl-6-[(2-thiényl)-carbonyl]-2--benzodioxole-carboxylique et ses sels de bases.

11. L'acide 5-benzoyl-6-fluoro-2-benzodioxole--carboxylique et ses sels de bases.

12. L'acide 5-benzoyl-6-chloro-2-benzodioxole--carboxylique et ses sels de bases.

13. Compositions pharmaceutiques contenant un composé selon l'une des revendications 1 à 12 ou un sel acceptable pour l'usage pharmaceutique d'un composé selon la revendication 1 apte à la salification ou d'un composé selon l'une des revendications 2 à 12.

14. Composés selon l'une des revendications 1 à 12 et ses sels acceptables pour l'usage pharmaceutique de composés selon la revendication 1 aptes à la salification ou de composés selon l'une des revendications 2 à 12 pour l'utilisation dans un procédé pour le traitement prophylactique et/ou thérapeutique de l'organisme animal ou humain.

15. Composés selon l'une des revendications 1 à 12 et sels acceptables pour l'usage pharmaceutique de composés selon la revendication 1 aptes à la salification ou d'un composés selon l'une des revendications 2 à 12 en tant que diurétiques à activité additionnelle uricosurique.

16. Utilisation des composés selon l'une des revendications 1 à 12, ou de sels acceptables pour l'usage pharmaceutique de composés selon la revendication 1 aptes à la salification ou d'un composé selon l'une des revendications 2 à 12 pour la préparation de médicaments par voie non chimique.

17. Procédé de préparation des dérivés du benzodioxole de formule générale I de la revendication 1, dans laquelle $R_1$, $R_2$, $R_3$, Alk, n et A ont les significations indiquées dans la revendication 1, et de sels de ces composés, caractérisé en ce que:

a) on fait réagir un composé de formule générale II:

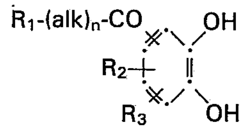

(II)

dans laquelle $R_1$, alk, n, $R_2$ et $R_3$ ont les significations indiquées dans la revendication 1, ou un sel d'un tel composé, avec un composé de formule générale III:

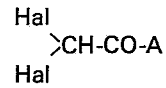

(III)

dans laquelle Hal représente un halogène et A a la signification indiquée dans la revendication 1, ou avec un sel d'un tel composé dans lequel A représente $OR_4$ et $R_4$ représente l'hydrogène, ou bien

b) dans un composé de formule générale IV:

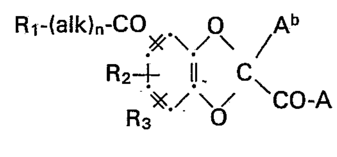

(IV)

dans laquelle $A^b$ représente un groupe carboxy, alcoxycarbonyle inférieur ou acétyle et $R_1$, alk, n, $R_2$, $R_3$ et A ont les significations indiquées dans la revendication 1, on remplace le groupe $A^b$ par l'hydrogène, ou bien

c) pour préparer un composé de formule générale I dans laquelle A a la signification indiquée dans la revendication 1 à l'exception d'un reste $OR_4$ dans lequel $R_4$ représente l'hydrogène, et $R_1$, alk, n, $R_2$ et $R_3$ ont les significations indiquées dans la revendication 1, on fait réagir un composés de formule générale V

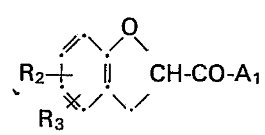

(V)

avec un anhydride d'un composé de formule générale VI:

$R_1$-(alk)$_n$-CO-OH (VI)

dans laquelle $A_1$ a la signification indiquée pour A dans la revendication 1, à l'exception d'un groupe $OR_4$ dans lequel $R_4$ représente l'hydrogène, et $R_2$ et $R_3$ d'une part, $R_1$, alk et n d'autre part, ont les significations indiquées dans la revendication 1, ou bien

d) pour préparer un composé de formule générale I dans laquelle A représente $OR_4$ dans lequel $R_4$ représente l'hydrogène, $R_1$, alk, n, $R_2$ et $R_3$ ayant les significations indiquées dans la revendication 1, ou un sel d'un tel composé, on part d'un composé de formule générale VII:

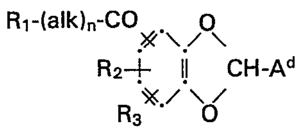

(VII)

dans laquelle $A^d$ représente un groupe convertible en le groupe carboxy, et $R_1$, alk, n, $R_2$ et $R_3$ ont les significations indiquées dans la revendication 1, et on convertit le groupe $A^d$ en le groupe carboxy libre ou à l'état de sel, ou bien

e) pour préparer un composé de formule générale I dans laquelle A a la signification indiquée dans la revendication 1, à l'exception d'un groupe $OR_4$ dans lequel $R_4$ représente l'hydrogène, et $R_1$, alk, n, $R_2$ et $R_3$ ont les significations indiquées dans la revendication 1, on part d'un composé de formule générale VIII:

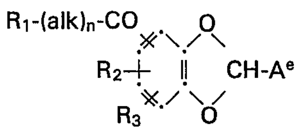

(VIII)

dans laquelle $A^e$ représente un reste convertible en un reste $CO-A_1$ dans lequel $A_1$ a la signification indiquée pour A dans la revendication 1, à l'exception d'un groupe $OR_4$ dans lequel $R_4$ représente l'hydrogène, et $R_1$, alk, n, $R_2$ et $R_3$ ont les significations indiquées dans la revendication 1, et on convertit le reste $A^e$ en le reste $-CO-A_1$, et si on le désire, on convertit un composé obtenu répondant à la formule générale I, de manière connue en soi, en un autre composé de formule générale I, et/ou convertit un composé obtenu, répondant à la formule générale I dans laquelle A représente $OR_4$ et $R_4$ l'hydrogène, en un sel à l'aide d'une base, ou bien on libère un tel composé à partir d'un sel obtenu, ou bien on convertit un composé répondant à la formule générale I et ayant un caractère basique en un sel par addition avec un acide ou bien on libère un tel composé à partir d'un sel obtenu.

18. Les composés de formule générale I de la revendication 1, dans lesquels $R_1$, alk, n, $R_2$, $R_3$ et A ont les significations indiquées dans la revendication 1, susceptibles d'être obtenus par les procédés de la revendication 17, et les sels des composés de formule générale I susceptibles d'être obtenus par ces procédés et qui sont aptes à une salification.

**Revendications pour l'Etat contractant:** AT

1. Procédé de préparation de dérivés du benzodioxole répondant à la formule générale I:

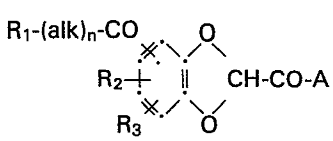

(I)

dans laquelle $R_1$ représente un reste aromatique ou hétéroaromatique non substitué ou substitué par des groupes alkyle inférieurs, alcoxy inférieurs, des halogènes ou des groupes trifluorométhyle, alk représente un groupe alkylène ou alkylidène à 5 atomes de carbone au maximum et n est égal à 0 ou 1, $R_2$ et $R_3$ représentent chacun indépendamment l'un de l'autre, l'hydrogène, un groupe alkyle inférieur, alcoxy inférieur ou un halogène et A représente le reste $-O-R_4$ dans lequel $R_4$ représente l'hydrogène ou un reste hydrocarboné aliphatique ou araliphatique non substitué ou substitué par des groupes alcoxy inférieurs ou des atomes d'halogènes, ou le reste:

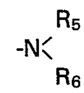

dans lequel $R_5$ et $R_6$ représentent chacun, indépendamment l'un de l'autre, l'hydrogène ou un groupe alkyle inférieur, ou bien forment ensemble et avec l'atome d'azote voisin un groupe tétra- à hexa-méthylène-imino ou 4-morpholinyle éventuellement substitué par des groupes alkyle inférieurs, et des sels de ces composés, l'expression «inférieur» s'appliquant à des groupes qui contiennent au maximum 7 atomes de carbone, caractérisé en ce que:

a) on fait réagir un composé de formule générale II:

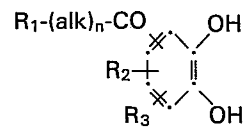

(II)

dans laquelle $R_1$, alk, n, $R_2$ et $R_3$ ont les significations indiquées en référence à la formule I, ou un sel d'un tel composé, avec un composé de formule générale III:

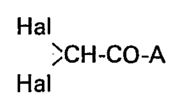

(III)

dans laquelle Hal représente un halogène et A a la signification indiquée en référence à la formule I, ou avec un sel d'un tel composé dans lequel A représente $OR_4$ et $R_4$ représente l'hydrogène, ou bien

b) dans un composé de formule générale IV:

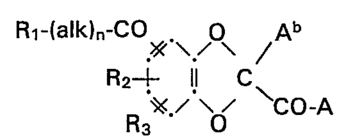

(IV)

dans laquelle $A^b$ représente un groupe carboxy, alcoxycarbonyle inférieur ou acétyle et $R_1$, alk, $R_2$, $R_3$ et A ont les significations indiquées en référence à la formule I, on remplace le groupe $A^b$ par l'hydrogène, ou bien

c) pour préparer un composé de formule générale I dans laquelle A a la signification indiquée en référence à la formule I à l'exception d'un groupe $OR_4$ dans lequel $R_4$ représente l'hydrogène, et $R_1$, alk, n, $R_2$ et $R_3$ ont les significations indiquées en référence à la formule I, on fait réagir un composés de formule générale V

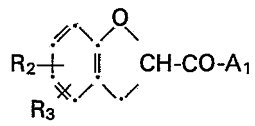

(V)

avec un anhydride d'un composé de formule générale VI:

$$R_1\text{-}(alk)_n\text{-}CO\text{-}OH \qquad (VI)$$

dans laquelle $Ar_1$ a la signification indiquée pour A en référence à la formule I, à l'exception d'un groupe $OR_4$ dans lequel $R_4$ représente l'hydrogène, et $R_2$ et $R_3$ d'une part, $R_1$, alk et n d'autre part, ont les significations indiquées en référence à la formule I, ou bien

d) pour préparer un composé de formule générale I dans laquelle A représente $OR_4$ dans lequel $R_4$ représente l'hydrogène, $R_1$, alk, n, $R_2$ et $R_3$ ayant les significations indiquées en référence à la formule I, ou un sel d'un tel composé, on part d'un composé de formule générale VII:

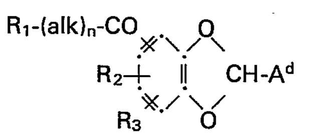

(VII)

dans laquelle $A^d$ représente un groupe convertible en le groupe carboxy, et $R_1$, alk, n, $R_2$ et $R_3$ ont les significations indiquées en référence à la formule I, et on convertit le groupe $A^d$ en le groupe carboxy libre ou à l'état de sel, ou bien

e) pour préparer un composé de formule générale I dans laquelle A a la signification indiquée en référence à la formule I, à l'exception d'un groupe $OR_4$ dans lequel $R_4$ représente l'hydrogène, et $R_1$, alk, n, $R_2$ et $R_3$ ont les significations indiquées en référence à la formule I, on part d'un composé de formule générale VIII:

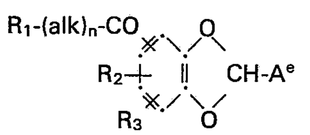

(VIII)

dans laquelle $A^e$ représente un groupe convertible en un groupe $CO\text{-}A_1$ dans lequel $A_1$ a la signification indiquée pour A en référence à la formule I, à l'exception d'un groupe $OR_4$ dans lequel $R_4$ représente l'hydrogène, et $R_1$, alk, n, $R_2$ et $R_3$ ont les significations indiquées en référence à la formule I, et on convertit le groupe $A^e$ en le groupe $\text{-}CO\text{-}A_1$, et si on le désire, on convertit un composé obtenu répondant à la formule générale I, de manière connue en soi, en un autre composé de formule générale I, et/ou on convertit un composé obtenu, répondant à la formule générale I dans laquelle A représente $OR_4$ et $R_4$ l'hydrogène, en un sel à l'aide d'une base, ou bien on libère un tel composé à partir d'un sel obtenu, ou bien on convertit un composé obtenu répondant à la formule générale I et ayant un caractère basique en un sel par addition avec un acide ou on libère un tel composé à partir d'un sel obtenu.

2. Procédé selon la revendication 1, caractérisé en ce que l'on prépare des composés de formule générale I de la revendication 1, dans lesquels $R_1$ représente un groupe phényle, thiényle ou furyle substitué par des groupes alkyle inférieurs, alcoxy inférieurs ou des halogènes, ou non substitué, alk représente un groupe alkylène ou alkylidène à 3 atomes de carbone au maximum, $R_2$ représente un groupe alkyle inférieur ou un halogène, $R_3$ représente l'hydrogène, un groupe alkyle inférieur ou un halogène et n et A ont les significations indiquées dans la revendication 1, à l'état de racémates ou d'antipodes optiques, ainsi que les sels de ces composés dans lesquels A représente $OR_4$ et $R_4$ l'hydrogène, et de bases, l'expression «inférieur» s'appliquant à des groupes qui contiennent au maximum 7 atomes de carbone.

3. Procédé selon la revendication 1, caractérisé en ce que l'on prépare des composés de formule générale I de la revendication 1, dans lesquels $R_1$ représente un groupe phényle ou thiényle substitué par des groupes alkyle inférieurs, alcoxy inférieurs ou des halogènes, ou non substitué, alk représente un groupe méthylène, n est égal à 0 ou 1, $R_2$ représente un groupe alkyl inférieur ou un halogène, $R_3$ représente l'hydrogène ou un groupe alkyle inférieur, et A représente $OR_4$ dans lequel $R_4$ représente l'hydrogène ou un groupe alkyle inférieur, à l'état de racémates ou d'antipodes optiques, ainsi que les sels acceptables pour l'usage pharmaceutique de ces composés dans lesquels $R_4$ représente l'hydrogène, l'expression «inférieur» s'appliquant à des groupes qui contiennent au maximum 7 atomes de carbone.

4. Procédé selon la revendication 1, caractérisé en ce que l'on prépare des composés de formule générale I de la revendication 1, dans lesquels $R_1$ représente un groupe phényle substitué par des groupes alkyle inférieurs, alcoxy inférieurs ou des halogènes ou non substitué, alk représente un groupe méthylène, n est égal à 0 ou 1, $R_2$ représente un groupe alkyle inférieur ou un halogène, $R_3$ représente l'hydrogène ou un groupe alkyle inférieur et A représente $OR_4$ dans lequel $R_4$ représente l'hydrogène ou un groupe alkyle inférieur, à l'état de racémates ou d'antipodes optiques, ainsi que les sels acceptables pour l'usage pharmaceutique de ces composés dans lesquels $R_4$ représente l'hydrogène, et de base, l'expression «inférieur» s'appliquant à des groupes qui contiennent au maximum 7 atomes de carbone.

5. Procédé selon la revendication 1, caractérisé en ce que l'on prépare des composés de formule générale I de la revendication 1, dans lesquels $R_1$ représente un groupe phényle substitué par des groupes alkyle inférieurs, alcoxy inférieurs ou des halogènes, ou non substitué, n est égal à 0, $R_2$ représente un groupe alkyle inférieur ou un halogène, $R_3$ représente l'hydrogène et A représente $OR_4$ dans lequel $R_4$ représente l'hydrogène ou un groupe alkyle inférieur, et le groupe acyle est fixé en position 5 ou 6, à l'état de racémates ou d'antipodes optiques, ainsi que les sels acceptables pour l'usage pharmaceutique de ces composés dans lesquels $R_4$ représente l'hydrogène, et de bases, l'expression «inférieur» s'appliquant à des groupes qui contiennent au maximum 7 atomes de carbone.

6. Procédé selon la revendication 1, caractérisé en ce que l'on prépare l'acide 5-benzoyl-6-méthyl--2-benzodioxole-carboxylique, ses antipodes optiques et ses sels de bases.

7. Procédé selon la revendication 1, caractérisé en ce que l'on prépare l'acide 5-(4-fluorobenzoyl)-6--méthyl-2-benzodioxole-carboxilique et ses sels de bases.

8. Procédé selon la revendication 1, caractérisé en ce que l'on prépare l'acide 5-(4-méthoxybenzoyl)-6-méthyl-2-benzodioxole-carboxylique et ses sels de bases.

9. Procédé selon la revendication 1, caractérisé en ce que l'on prépare l'acide 5-[(4-fluorophényl)--acétyl]-6-méthyl-2-benzodioxole-carboxylique et ses sels de bases.

10. Procédé selon la revendication 1, caractérisé en ce que l'on prépare l'acide 5-méthyl-6-[(2-thiényl)-carbonyl]-2-benzodioxole-carboxylique et ses sels de bases.

11. Procédé selon la revendication 1, caractérisé en ce que l'on prépare l'acide 5-benzoyl-6-fluoro-2--benzodioxole-carboxylique et ses sels de bases.

12. Procédé selon la revendication 1, caractérisé en ce que l'on prépare l'acide 5-benzoyl-6-chloro-2--benzodioxole-carboxylique et ses sels de bases.